# EUROPEAN PATENT SPECIFICATION

(11) **EP 4 524 886 B1**
(45) Date of publication and mention of the grant of the patent: **13.05.2026**
(21) Application number: 24193760.6
(22) Date of filing: 09.08.2024
(51) Int. Cl.: G06T 7/00

(54) **LESION DETECTION METHOD AND LESION DETECTION PROGRAM**
LÄSIONSDETEKTIONSVERFAHREN UND LÄSIONSDETEKTIONSPROGRAMM
PROCÉDÉ ET PROGRAMME DE DÉTECTION DE LÉSION

(30) Priority: 13.09.2023 JP 2023148663
(43) Date of publication of application: 19.03.2025
(73) Proprietor: Fujitsu Limited, Kawasaki-shi, Kanagawa 211-8588 (JP)
(72) Inventor: Baba, Kozo, Kawasaki-shi, Kanagawa 211-8588 (JP); Takebe, Hiroaki, Kawasaki-shi, Kanagawa 211-8588 (JP); Miyazaki, Nobuhiro, Kawasaki-shi, Kanagawa 211-8588 (JP)
(74) Representative: Hoffmann Eitle

(56) References cited:
- CN-A- 109 635 664
- US-B2- 9 996 922
- GYORFI AGNES ET AL: "Brain Tumor Detection and Segmentation from Magnetic Resonance Image Data Using Ensemble Learning Methods", 2019 IEEE INTERNATIONAL CONFERENCE ON SYSTEMS, MAN AND CYBERNETICS (SMC), IEEE, 6 October 2019 (2019-10-06), pages 909 - 914, XP033667947, DOI: 10.1109/SMC.2019.8914463
- RAFID A. K. ET AL: "An Effective Ensemble Machine Learning Approach to Classify Breast Cancer Based on Feature Selection and Lesion Segmentation Using Preprocessed Mammograms", BIOLOGY, vol. 11, no. 1654, 11 November 2022 (2022-11-11), CH, XP093236003, ISSN: 2079-7737, DOI: 10.3390/biology11111654
- BRUNESE LUCA ET AL: "An ensemble learning approach for brain cancer detection exploiting radiomic features", COMPUTER METHODS AND PROGRAMS IN BIOMEDICINE, ELSEVIER, AMSTERDAM, NL, vol. 185, 22 October 2019 (2019-10-22), XP086049624, ISSN: 0169-2607, [retrieved on 20191022], DOI: 10.1016/J.CMPB.2019.105134
- MAHMOOD QAISER ET AL: "Automatic Ischemic Stroke Lesion Segmentation in Multi-spectral MRI Images Using Random Forests Classifier", 5 October 2015, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 266 - 274, ISBN: 978-3-540-74549-5, XP047358820

## Description

### FIELD

The embodiments discussed herein are related to a lesion detection method and a non-transitory computer-readable recording medium storing a lesion detection program.

### BACKGROUND

Medical images obtained by computed tomography (CT), magnetic resonance imaging (MRI), or the like are widely used for diagnosis of various diseases. For image diagnosis using the medical images, a doctor has to interpret a large number of images, which imposes a large burden on the doctor. For this reason, there is a demand for a technique for supporting diagnosis work of the doctor by a computer in some form.

As an example of such a technique, there is a technique for detecting a lesion region from a medical image by using a trained model generated by machine learning. For example, an artificial intelligence pipeline for lesion detection and classification including a plurality of trained machine learning models is proposed.

Prior art document D1, "Brain Tumor Detection and Segmentation from Magnetic Resonance Image Data Using Ensemble Learning Methods", GYORFI AGNES, XP033667947 refers to an evaluation framework designed to test the accuracy and efficiency of ensemble learning algorithms deployed for brain tumor segmentation using the BraTS 2016 train data set. Within this category of machine learning algorithms, random forest was found the most appropriate, both in terms of precision and runtime.

Prior art document D2, CN 109 635 664 A relates to a fatigue driving detection method based on illumination detection, and belongs to the field of computer detection. It proposes a fatigue driving detection method based on illumination detection. In order to solve the above problems, in the training process, the collected pictures are classified according to the illumination intensity, and the picture sets of different illumination intensities are separately trained, respectively. Corresponding classification model; in the prediction process, according to the current illumination intensity, it is judged which classification model needs to be used for prediction, so that the classification is more refined, so that the detection accuracy is higher.

### Citation List

### Patent Literature

U.S. Patent Application Publication No. 2022/0270254

### TECHNICAL PROBLEM

Meanwhile, in the detection process of the lesion region using the trained model, it is possible to increase detection accuracy as a difference in pixel value (for example, luminance value) between the lesion region and a normal region that is not a lesion is larger. Meanwhile, there are many cases where the difference in pixel value between the lesion region and the normal region is small in an actually captured medical image, and for this reason, it is difficult to increase the detection accuracy, in some cases. In a case where a medical event that may affect the pixel value such as fat accumulated in an organ occurs, the difference in pixel value between the lesion region and the normal region may be smaller than usual. In some cases, a magnitude relationship in pixel value between the lesion region and the normal region may be reversed.

According to one aspect, an object of the present disclosure is to provide a lesion detection method and a lesion detection program capable of improving detection accuracy of a lesion region from a medical image.

### SOLUTION TO PROBLEM

This object is accomplished by the subject-matter of the independent claims. The dependent claims concern particular embodiments.

The object and advantages of the invention will be realized and attained by means of the elements and combinations particularly pointed out in the claims.

It is to be understood that both the foregoing general description and the following detailed description are exemplary and explanatory and are not restrictive of the invention.

### EFFECTS OF INVENTION

According to one aspect, detection accuracy of a lesion region from a medical image is improved.

### BRIEF DESCRIPTION OF DRAWINGS

FIG. 1 is a diagram illustrating a configuration example and a process example of an information processing apparatus according to a first embodiment;
FIG. 2 is a diagram illustrating a configuration example of a diagnosis support system according to a second embodiment;
FIG. 3 is a diagram illustrating an example of a machine learning model for detecting a lesion region;
FIG. 4 is a diagram illustrating a configuration example of a processing function provided by a diagnosis support apparatus;
FIG. 5 is a diagram illustrating a data configuration example of a learning data set;
FIG. 6 is a diagram for describing a generation process of a lesion identification model;
FIG. 7 is a diagram for describing a lesion detection process using the lesion identification model;
FIG. 8 is an example of a weight table indicating a correspondence relationship between an image feature amount and a weight coefficient;
FIG. 9 is an example of a flowchart illustrating a procedure of a model generation process;
FIG. 10 is an example of a flowchart illustrating a procedure of the lesion detection process;
FIG. 11 is a diagram illustrating a screen display example of a detection result of the lesion region;
FIG. 12 is a diagram illustrating a configuration example of a processing function provided by a diagnosis support apparatus according to a third embodiment;
FIG. 13 is a diagram for describing the generation process of the lesion identification model;
FIG. 14 is an example of another weight table indicating the correspondence relationship between the image feature amount and the weight coefficient;
FIG. 15 is an example of another flowchart illustrating the procedure of the model generation process;
FIG. 16 is an example of another flowchart illustrating the procedure of the lesion detection process;
FIG. 17 is a diagram for describing a model generation process of a diagnosis support apparatus according to a fourth embodiment;
FIG. 18 is an example of still another weight table indicating the correspondence relationship between the image feature amount and the weight coefficient;
FIG. 19 is an example of still another flowchart illustrating the procedure of the model generation process;
FIG. 20 is an example of still another flowchart illustrating the procedure of the lesion detection process; and
FIG. 21 is an example of still another weight table indicating the correspondence relationship between the image feature amount and the weight coefficient.

### DESCRIPTION OF EMBODIMENTS

Hereinafter, embodiments of the present disclosure will be described with reference to the drawings.

### [First Embodiment]

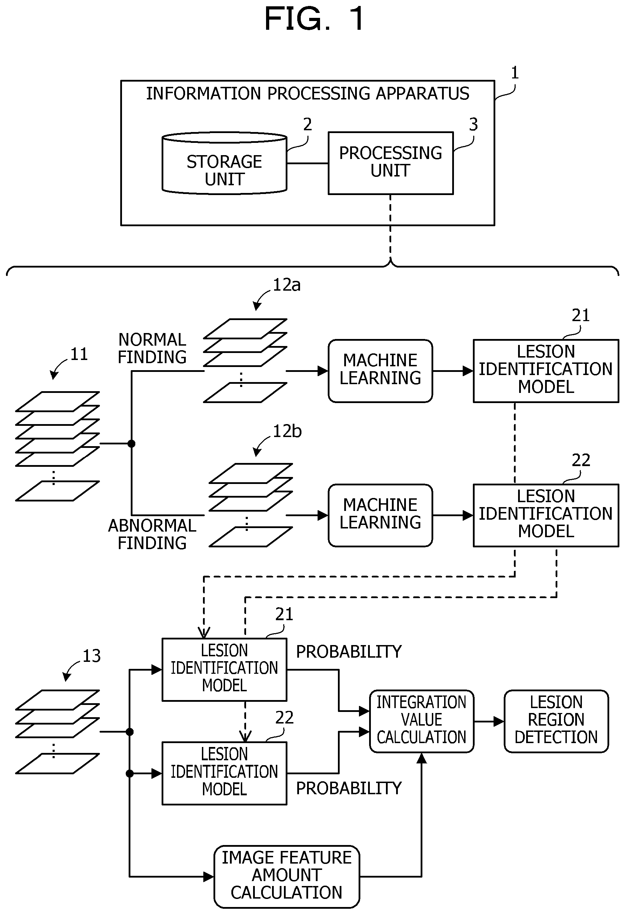

FIG. 1 is a diagram illustrating a configuration example and a process example of an information processing apparatus according to a first embodiment. An information processing apparatus 1 illustrated in FIG. 1 detects a specific lesion region from a tomographic image obtained by imaging an inside of a human body. As an example in the following description, it is assumed that the tomographic image is an image captured at certain intervals in a height direction of the human body. For the detection of the lesion region, a lesion identification model for identifying whether or not each unit image region included in the input tomographic image is a lesion region is used. The unit image region is, for example, a pixel over a tomographic image or a set (image block) of the predetermined number of two or more adjacent pixels. The information processing apparatus 1 may also generate such a lesion identification model by machine learning.

The information processing apparatus 1 includes a storage unit 2 and a processing unit 3. The storage unit 2 is a storage region secured in a storage device (not illustrated) included in the information processing apparatus 1. For example, a model parameter indicating a lesion identification model is stored in the storage unit 2. The processing unit 3 is, for example, a processor. In this case, the following process of the processing unit 3 is realized by, for example, the processor executing a predetermined program. The processing unit 3 executes a learning process of generating a lesion identification model by machine learning and a lesion detection process of detecting a lesion region by using the generated lesion identification model.

A process to be executed in the learning process is as follows. The processing unit 3 classifies a plurality of first tomographic images obtained by imaging insides of a plurality of first human bodies into a plurality of tomographic image groups having different medical findings. The processing unit 3 generates a plurality of lesion identification models for identifying whether or not each unit image region included in a tomographic image of an identification target is a lesion region by machine learning using each of ones different from each other among the plurality of tomographic image groups as learning data.

As an example in FIG. 1, a tomographic image group 11 including the plurality of first tomographic images described above is prepared, and the first tomographic images included in the tomographic image group 11 are classified into two tomographic image groups 12a and 12b having different medical findings. As the medical finding, for example, a finding related to an abnormal event that may change a relationship of pixel values between a lesion region and a normal region in a tomographic image is used. As the example in FIG. 1, it is assumed that the first tomographic images included in the tomographic image group 11 are classified into the tomographic image group 12a of a normal finding in which an abnormal event does not occur and the tomographic image group 12b of an abnormal finding in which an abnormal event occurs. Actually, the medical finding may be determined by using the plurality of first tomographic images obtained by one imaging for each person as a unit.

By machine learning using the tomographic image included in the tomographic image group 12a as learning data, the processing unit 3 generates a lesion identification model 21. The processing unit 3 generates a lesion identification model 22 by machine learning using the tomographic image included in the tomographic image group 12b as learning data.

As the abnormal event as described above, for example, accumulation of fat in an organ such as a liver may be applied. A tumor in an organ region is imaged darker than a normal organ region (normal region) in a CT image. On the other hand, since a fat region also appears dark, when fat is accumulated in an organ, the entire CT image is dark, and a difference in brightness between a lesion region (tumor region) and a normal region is small. In some cases, light and dark are reversed between the lesion region and the normal region. In this manner, there is a possibility that a relationship between pixel values of the lesion region and the normal region in the CT image is changed depending on whether fat is accumulated or not.

For such a problem, the lesion identification model 21 appropriate for a tomographic image of a normal finding and the lesion identification model 22 appropriate for a tomographic image of an abnormal finding are generated by the process described above of the processing unit 3. For example, as compared with a lesion identification model generated by using a CT image group in which a normal finding CT image in which fat is not accumulated and an abnormal finding CT image in which fat is accumulated are mixed, the lesion identification model 21 may detect a lesion region from the tomographic image of normal finding with high accuracy. On the other hand, the lesion identification model 22 may detect a lesion region from the tomographic image of the abnormal finding with high accuracy, as compared with a lesion identification model generated by using the CT image group in which the normal finding CT image and the abnormal finding CT image are mixed.

After the lesion identification models 21 and 22 are generated as described above, in the lesion process, the following process is executed. The processing unit 3 acquires a tomographic image group 13 including a plurality of second tomographic images obtained by imaging an inside of a second human body. Based on the acquired tomographic image group 13, the processing unit 3 calculates an image feature amount. For example, in a case where the abnormal event as described above is applied, an average luminance in an organ region of each second tomographic image included in the tomographic image group 13 may be used as the image feature amount.

The processing unit 3 inputs the plurality of second tomographic images included in the tomographic image group 13 to each of the generated lesion identification models 21 and 22. Thus, an identification process for a lesion region is executed by each of the lesion identification models 21 and 22. The processing unit 3 acquires a probability that each unit image region included in the plurality of second tomographic images is a lesion region, from the lesion identification model 21. The processing unit 3 also acquires a probability that each unit image region included in the plurality of second tomographic images is a lesion region, from the lesion identification model 22.

For each unit image region included in the plurality of second tomographic images, the processing unit 3 integrates the probabilities acquired from the lesion identification models 21 and 22, based on the calculated image feature amount to calculate an integration value. Based on the calculated integration value, the processing unit 3 detects a lesion region from each of the plurality of second tomographic images. For example, in a case where the integration value is equal to or more than a predetermined threshold value, the corresponding unit image region is identified as a lesion region, and in a case where the integration value is less than the threshold value, the corresponding unit image region is identified as a not-lesion region.

With the process described above, for example, the occurrence degree of abnormal event (accumulation degree of fat) in the plurality of second tomographic images is estimated based on the image feature amount. By calculating the integration value, the processing unit 3 may give a larger weight to a probability from an appropriate lesion identification model in accordance with the occurrence degree of abnormal event, and perform weighting addition on the probability. For example, since the tomographic image is estimated as a normal finding in a case where the image feature amount (average luminance) is equal to or more than a predetermined threshold value, the lesion identification model 22 gives a large weight to the probability by the lesion identification model 21. On the other hand, since the tomographic image is estimated as an abnormal finding in a case where the image feature amount (average luminance) is less than the threshold value, the lesion identification model 21 gives a large weight to the probability by the lesion identification model 22.

With such a process, regardless of the finding related to the abnormal event described above (for example, regardless of the presence or absence of accumulation of fat) for the plurality of input second tomographic images, the processing unit 3 may detect a lesion region from each second tomographic image with high accuracy. Accordingly, the detection accuracy of the lesion region from the tomographic image may be improved.

### [Second Embodiment]

Next, a system capable of detecting a lesion region of a liver from a CT image will be described.

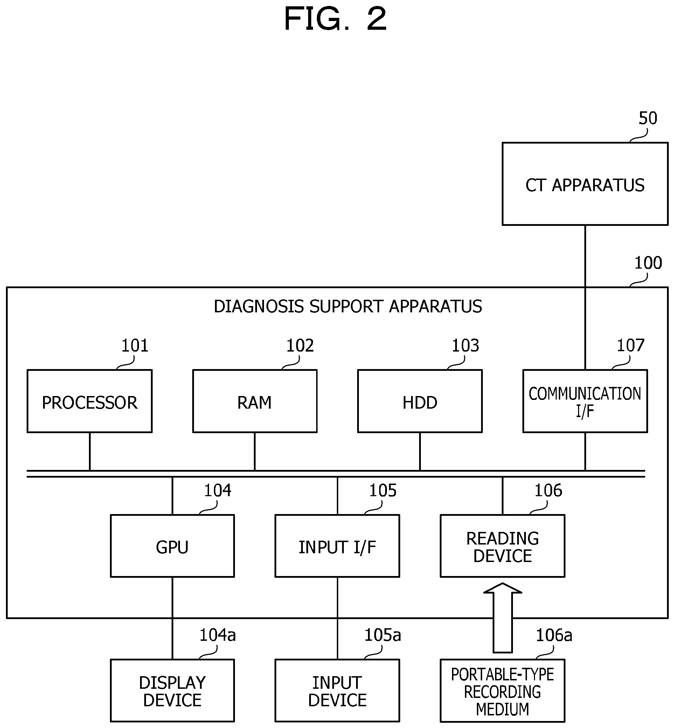

FIG. 2 is a diagram illustrating a configuration example of a diagnosis support system according to a second embodiment. The diagnosis support system illustrated in FIG. 2 is a system that supports image diagnosis work by CT imaging, and includes a CT apparatus 50 and a diagnosis support apparatus 100.

The CT apparatus 50 captures an X-ray CT image of a human body. According to the present embodiment, the CT apparatus 50 captures a predetermined number of tomographic images of an axial plane in an abdominal region including the liver while changing a position (slice position) of the human body in a height direction (direction perpendicular to the axial plane) at a predetermined interval.

The diagnosis support apparatus 100 extracts a liver region from each tomographic image captured by the CT apparatus 50, and detects a lesion region based on image information on the extracted liver region. According to the present embodiment, it is assumed that a tumor (for example, a hemangioma) in the liver is detected as a lesion region. For example, the diagnosis support apparatus 100 causes a display device to display information indicating a detection result of the lesion region. Thus, the diagnosis support apparatus 100 supports the image diagnosis work of a user (for example, a radiologist).

The diagnosis support apparatus 100 detects a lesion region (or identifies a lesion) by using a trained model generated by machine learning. The diagnosis support apparatus 100 is also capable of executing a process of generating the trained model by machine learning.

A hardware configuration of the diagnosis support apparatus 100 will be described below with reference to FIG. 2. The diagnosis support apparatus 100 is realized as a computer illustrated in FIG. 2, for example. As illustrated in FIG. 2, the diagnosis support apparatus 100 includes a processor 101, a random-access memory (RAM) 102, a hard disk drive (HDD) 103, a graphics processing unit (GPU) 104, an input interface (I/F) 105, a reading device 106, and a communication interface (I/F) 107.

The processor 101 comprehensively controls the entire diagnosis support apparatus 100. The processor 101 is, for example, a central processing unit (CPU), a microprocessor unit (MPU), a digital signal processor (DSP), an application-specific integrated circuit (ASIC), or a programmable logic device (PLD). The processor 101 may also be a combination of two or more elements among the CPU, the MPU, the DSP, the ASIC, and the PLD. The processor 101 is an example of the processing unit 3 illustrated in FIG. 1.

The RAM 102 is used as a main storage device of the diagnosis support apparatus 100. The RAM 102 temporarily stores at least a part of an operating system (OS) program and an application program to be executed by the processor 101. The RAM 102 also stores various types of data to be used in processes performed by the processor 101.

The HDD 103 is used as an auxiliary storage device of the diagnosis support apparatus 100. The OS program, the application program, and various types of data are stored in the HDD 103. That a different type of non-volatile storage device such as a solid-state drive (SSD) may be used as the auxiliary storage device.

A display device 104a is coupled to the GPU 104. The GPU 104 causes the display device 104a to display an image in accordance with an instruction from the processor 101. Examples of the display device 104a include a liquid crystal display, an organic electroluminescence (EL) display, and the like.

An input device 105a is coupled to the input interface 105. The input interface 105 transmits a signal output from the input device 105a to the processor 101. The input device 105a includes a keyboard, a pointing device, and the like. The pointing device includes a mouse, a touch panel, a tablet, a touch pad, a track ball, and the like.

A portable-type recording medium 106a is removably attached to the reading device 106. The reading device 106 reads data recorded in the portable-type recording medium 106a, and transmits the data to the processor 101. The portable-type recording medium 106a includes an optical disc, a semiconductor memory, and the like.

The communication interface 107 transmits and receives data to and from another apparatus such as the CT apparatus 50 via a network.

The hardware configuration as described above is capable of realizing a processing function of the diagnosis support apparatus 100.

As described above, the lesion region detection process from a medical image may be executed by using a trained model generated by machine learning, for example.

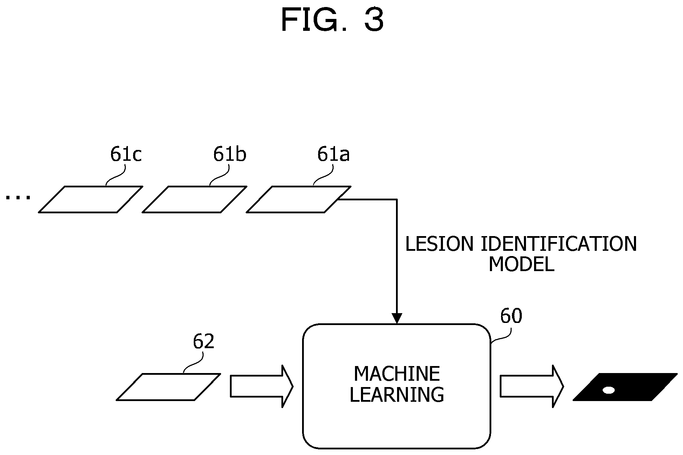

FIG. 3 is a diagram illustrating an example of a machine learning model for detecting a lesion region. A lesion identification model 60 illustrated in FIG. 3 is a trained model that identifies whether or not each pixel of a tomographic image 62 is a specific lesion region when a tomographic image 62 is input. Here, it is assumed that the tomographic image 62 is a tomographic image of an axial plane. Actually, the lesion identification model 60 calculates a probability of being a lesion region for each pixel of the input tomographic image 62, and identifies a pixel having a probability equal to or more than a predetermined value as the lesion region.

The lesion identification model 60 is generated by machine learning (for example, deep learning) using tomographic images 61a, 61b, 61c, and ... of the same axial plane as training data. Labels indicating whether or not each pixel is a lesion region are added to the tomographic images 61a, 61b, 61c, and ..., and these labels are used as correct answer data in the machine learning.

By using such a lesion identification model 60, the diagnosis support apparatus 100 according to the present embodiment may detect a lesion region. As another example of the lesion identification model, there is a model that identifies whether or not a partial image is a lesion region in units of partial images (patch images) having a certain size. In this case, each tomographic image used as learning data is divided into patch images having a certain size, and a label indicating whether or not the patch image is a lesion region is added to each of the divided patch images. By performing machine learning in units of patch images by using each patch image as training data and using the label as correct answer data, a lesion identification model is generated. At a time of inference, a tomographic image input to the lesion identification model is divided into patch images having a certain size, and the lesion identification model identifies whether or not each patch image is a lesion region.

Meanwhile, in the lesion identification process using the lesion identification model as described above, it is considered that, as a luminance difference between a lesion region and a normal region that is not a lesion in an organ region over a CT image is larger, identification accuracy of the lesion region is higher. At an actual medical site, CT imaging using a contrast agent is performed to emphasize a luminance difference between a lesion region and a normal region. Meanwhile, in some cases, side effects may occur in a diagnosis target person due to the use of the contrast agent. Therefore, the diagnosis target person who may use the contrast agent is limited to a diagnosis target person having a relatively good condition, such as a stage in which there is no subjective symptom due to a disease or a stage in which a disease is suspected.

For this reason, it is desirable that a lesion region is identified with high accuracy from a CT image (referred to as a "simple CT image" herein) captured without using the contrast agent. The simple CT image is often captured at a stage in which a specific disease is not suspected, such as in a medical examination. Detecting a serious disease such as cancer from the CT image captured at such a stage leads to early detection of a disease in a true sense. When a lesion region may be automatically and accurately identified from the simple CT image, it is possible to find a disease even in a medical institution in which a medical specialist for image diagnosis does not reside.

Meanwhile, in the simple CT image, in some cases, a luminance difference between a lesion region and a normal region in an organ region is small, and the lesion region may not be identified with high accuracy. For example, in a case where a tumor is detected from a liver, a luminance value of a tumor region is less than a luminance value of a normal region in the liver in the simple CT image, so that the tumor region appears darker. Meanwhile, in a case where fat is accumulated in the liver, the fat is also imaged dark over the simple CT image, so that the luminance value of the entire liver region is decreased. As a result, the luminance difference between the tumor region and the normal region is decreased, and it becomes difficult to identify the tumor region with high accuracy. Depending on another case, a magnitude relationship of luminance is reversed between the tumor region and the normal region, and it becomes more difficult to identify the tumor region with high accuracy.

Considering such a point, it is considered to be difficult to generate a lesion identification model with high identification accuracy in a case where a simple CT image including fat and a simple CT image not including fat are mixed in simple CT images used as learning data at a time of machine learning. This is because the machine learning is performed by using a simple CT image in which a luminance difference between a lesion region and a normal region varies or a simple CT image in which a magnitude relationship of luminance differs between the lesion region and the normal region.

Accordingly, in the present embodiment, a CT image group by simple CT images used as learning data is classified into a plurality of CT image groups in which the degrees of occurrence of abnormal events that may change the luminance relationship between the lesion region and the normal region are different from each other. In the example described above, accumulation of fat is the abnormal event. The classification of the CT image group in this manner may be performed based on findings by a radiologist.

The diagnosis support apparatus 100 according to the present embodiment generates a plurality of lesion identification models by using, as learning data, each of one CT image groups different from each other among the plurality of CT image groups classified in this manner. Thus, the plurality of lesion identification models corresponding to differences in features of input CT images are generated.

At a time of inference, the diagnosis support apparatus 100 acquires a CT image group of simple CT images, and estimates the occurrence degree of abnormal event in the acquired CT image group from an image feature amount of the CT image group. At the same time, the diagnosis support apparatus 100 executes a lesion identification process on the CT image group by using each of the plurality of lesion identification models described above, and acquires a probability value indicating a lesion region from each lesion identification model for each pixel of the CT image included in the CT image group. By integrating the acquired probability values at a ratio corresponding to the image feature amount, the diagnosis support apparatus 100 outputs a final identification result.

By integrating the probability values, it is possible to determine the final identification result by increasing the ratio of the probability values from the lesion identification model appropriate for the occurrence degree of abnormal event in the CT image group based on the image feature amount. The final identification result may be determined by selecting a probability value from one lesion identification model optimum for the occurrence degree of abnormal event in the CT image group. Therefore, it is possible to improve accuracy of lesion identification from the simple CT image.

A case where a lesion identification model generated by using a CT image of an abnormal finding in which fat is accumulated and a lesion identification model generated by using a CT image of a normal finding in which fat is not accumulated are mainly used will be exemplified in the following description.

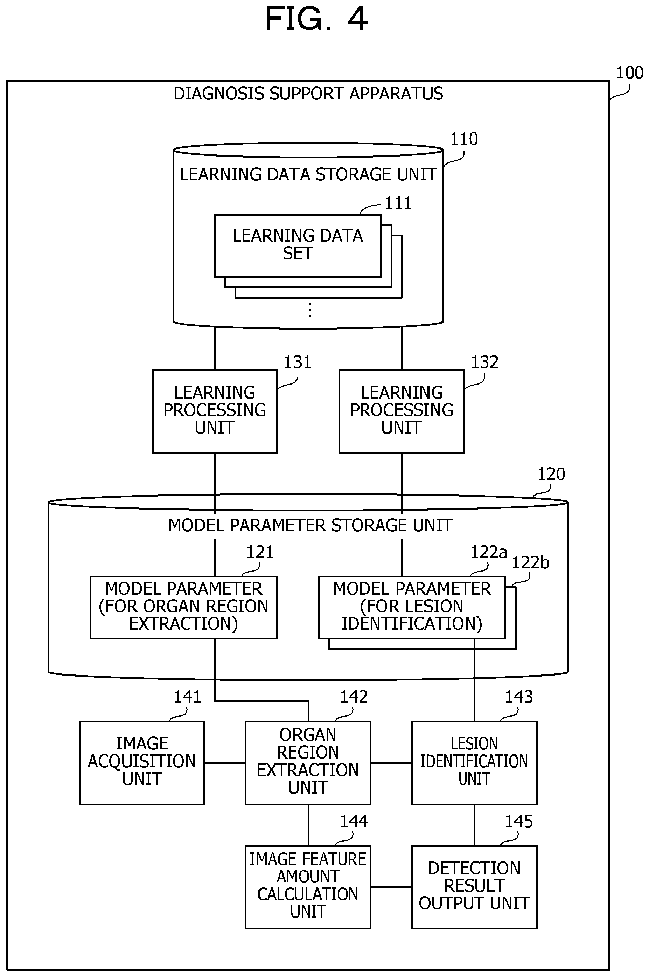

FIG. 4 is a diagram illustrating a configuration example of a processing function provided by a diagnosis support apparatus. The diagnosis support apparatus 100 includes a learning data storage unit 110, a model parameter storage unit 120, learning processing units 131 and 132, an image acquisition unit 141, an organ region extraction unit 142, a lesion identification unit 143, an image feature amount calculation unit 144, and a detection result output unit 145.

The learning data storage unit 110 and the model parameter storage unit 120 are storage regions secured in a storage device included in the diagnosis support apparatus 100, such as the RAM 102 or the HDD 103.

A plurality of learning data sets 111 to be used for generating a lesion identification model by machine learning are stored in the learning data storage unit 110. A CT image (tomographic image) set for one person as an imaging target is included in the learning data set 111. Examples of the learning data set 111 include a data set including a CT image set of a normal finding and a data set including a CT image set of an abnormal finding (finding that there is fat). By using these learning data sets 111, the diagnosis support apparatus 100 generates two lesion identification models corresponding to a difference in findings. any CT image included in each of the learning data sets 111 is a simple CT image captured without using a contrast agent.

The model parameter storage unit 120 stores a model parameter 121 corresponding to an organ region extraction model which is a trained model for extracting an organ region (liver region), and model parameters 122a and 122b corresponding to the two lesion identification models described above, respectively. A model parameter stored in the model parameter storage unit 120 is data for forming the trained model, and includes, for example, a weight coefficient between nodes over a neural network.

Processes of the learning processing units 131 and 132, the image acquisition unit 141, the organ region extraction unit 142, the lesion identification unit 143, the image feature amount calculation unit 144, and the detection result output unit 145 are realized by, for example, the processor 101 executing an application program.

The learning processing units 131 and 132 operate in a learning phase in which the organ region extraction model and the lesion identification model are to be generated by machine learning.

By performing machine learning using each learning data set 111 stored in the learning data storage unit 110, the learning processing unit 131 generates the organ region extraction model. The learning processing unit 131 stores the model parameter 121 indicating the generated organ region extraction model in the model parameter storage unit 120.

By performing machine learning using each learning data set 111 stored in the learning data storage unit 110, the learning processing unit 132 generates the lesion identification model. At this time, the learning processing unit 132 generates the lesion identification model corresponding to a normal finding by using the learning data set 111 (learning data set of the normal finding) including a CT image set of the normal finding. Along with this, the learning processing unit 132 generates the lesion identification model corresponding to an abnormal finding by using the learning data set 111 including a CT image set of the abnormal finding (learning data set of the abnormal finding). The learning processing unit 132 stores the model parameters 122a and 122b respectively corresponding to the two generated lesion identification models in the model parameter storage unit 120.

The image acquisition unit 141, the organ region extraction unit 142, the lesion identification unit 143, the image feature amount calculation unit 144, and the detection result output unit 145 operate in an inference phase in which a lesion region is detected by using the generated organ region extraction model and lesion identification model.

The image acquisition unit 141 acquires a CT image set obtained by imaging for a diagnosis target person from the CT apparatus 50. The acquired CT image set is an image set of simple CT images captured without using a contrast agent.

By using the organ region extraction model based on the model parameter 121, the organ region extraction unit 142 extracts a CT image including an organ region (liver region) from the CT image set.

For each of the CT images including the organ region, the lesion identification unit 143 executes a lesion identification process by using a lesion identification model based on the model parameter 122a, and outputs a probability value indicating a lesion region for each pixel. For each of the CT images including the organ region, the lesion identification unit 143 executes the lesion identification process by using a lesion identification model based on the model parameter 122b, and outputs a probability value indicating a lesion region for each pixel.

The image feature amount calculation unit 144 calculates an image feature amount from the organ region among image regions of the CT images included in the CT image set. According to the present embodiment, an average luminance in the organ region is calculated as the image feature amount.

The detection result output unit 145 integrates the lesion identification results by the two lesion identification models, and outputs a final detection result of the lesion region. For example, for each pixel of the CT image including the organ region, the detection result output unit 145 performs weighting addition on the probability value calculated by the two lesion identification models by using a weight coefficient according to the calculation result of the image feature amount. The detection result output unit 145 compares a calculated value by the weighting addition with a predetermined threshold value, and identifies a pixel having a calculated value equal to or more than the threshold value as a pixel of a lesion region.

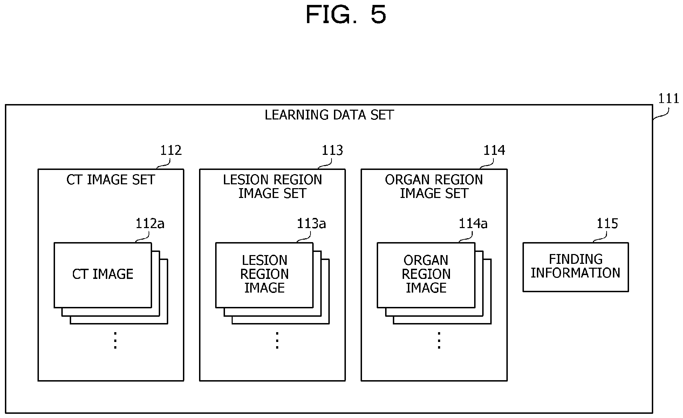

FIG. 5 is a diagram illustrating a data configuration example of a learning data set. The learning data set 111 stored in the learning data storage unit 110 includes a CT image set 112, a lesion region image set 113, an organ region image set 114, and finding information 115.

The CT image set 112 includes a plurality of CT images 112a (tomographic images) obtained by one imaging for one person as an imaging target.

The lesion region image set 113 includes lesion region images 113a respectively corresponding to the plurality of CT images 112a included in the CT image set 112. The lesion region image 113a is an image in which a label indicating whether or not each pixel of the corresponding CT image 112a is a lesion region is associated with the pixel. Each label of the lesion region image 113a is used as a correct answer label when a lesion identification model is generated by machine learning.

The organ region image set 114 includes organ region images 114a respectively corresponding to the plurality of CT images 112a included in the CT image set 112. The organ region image 114a is an image in which a label indicating whether or not each pixel of the corresponding CT image 112a is an organ region (liver region) is associated with the pixel. Each label of the organ region image 114a is used as a correct answer label when an organ region extraction model is generated by machine learning.

The finding information 115 is information indicating whether the plurality of CT images 112a included in the CT image set 112 have an abnormal finding or a normal finding. The finding information 115 is generated in units of the CT image set 112. For example, when a radiologist comprehensively views the CT image 112a included in the CT image set 112, in a case where it is determined that fat is accumulated in an organ to some extent or more, the CT image 112a is regarded as an abnormal finding, and in a case where it is determined that fat is not accumulated in the organ, the CT image 112a is regarded as a normal finding. In this manner, the finding information 115 is information generated based on the medical findings made by the radiologist.

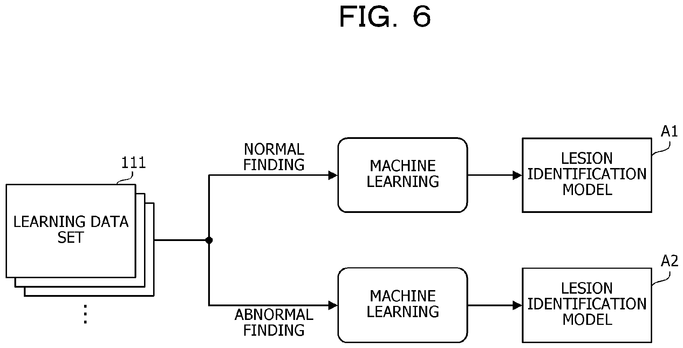

FIG. 6 is a diagram for describing a generation process of a lesion identification model. Based on the finding information 115, the learning data sets 111 stored in the learning data storage unit 110 are classified into the learning data set 111 including the CT image set 112 of a normal finding (the learning data set 111 of the normal finding) and the learning data set 111 including the CT image set 112 of an abnormal finding (the learning data set 111 of the abnormal finding).

Based on the finding information 115, the learning processing unit 132 extracts the learning data set 111 of the normal finding, from the learning data sets 111 stored in the learning data storage unit 110. By performing machine learning using each of the CT image 112a and the lesion region image 113a included in the extracted learning data set 111, the learning processing unit 132 generates a lesion identification model A1 corresponding to the normal finding. Actually, among the CT images 112a included in the CT image set 112 of the normal finding, the CT image 112a including an organ region and the lesion region image 113a corresponding to the CT image 112a are used for the machine learning. Thus, the model parameter 122a indicating the lesion identification model A1 is stored in the model parameter storage unit 120.

The learning processing unit 132 extracts the learning data set 111 having an abnormal finding based on the finding information 115, from the learning data sets 111 stored in the learning data storage unit 110. By performing machine learning using each of the CT image 112a and the lesion region image 113a included in the extracted learning data set 111, the learning processing unit 132 generates a lesion identification model A2 corresponding to the abnormal finding. Actually, among the CT images 112a included in the CT image set 112 of the abnormal finding, the CT image 112a including an organ region and the lesion region image 113a corresponding to the CT image 112a are used for the machine learning. Thus, the model parameter 122b indicating the lesion identification model A2 is stored in the model parameter storage unit 120.

With the above model generation process, the lesion identification model A1 appropriate for lesion detection from the CT image group in which fat is not accumulated in the organ region and the lesion identification model A2 appropriate for lesion detection from the CT image group in which fat is included in the organ region are individually generated.

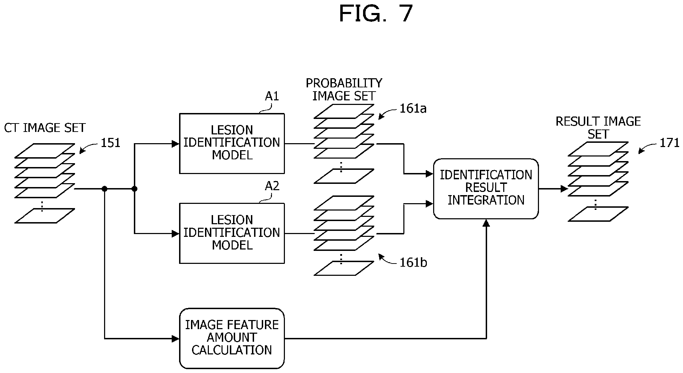

FIG. 7 is a diagram for describing a lesion detection process using a lesion identification model.

A diagnosis target person is captured by the CT apparatus 50, and a CT image set 151 is obtained. CT images including an organ region are extracted from the CT image set 151 by using an organ region extraction model, and each of the extracted CT images is input to both of the lesion identification models A1 and A2.

The lesion identification model A1 executes a lesion identification process in pixel units on each input CT image, and outputs a probability value indicating a probability of being a lesion region for each pixel. Thus, for example, a probability image set 161a including probability images respectively corresponding to the input CT images is generated. The probability image included in the probability image set 161a is an image in which a probability value is associated with each pixel of a corresponding CT image.

The lesion identification model A2 executes a lesion identification process in pixel units on each input CT image, and outputs a probability value indicating a probability of being a lesion region for each pixel. Thus, for example, a probability image set 161b including probability images respectively corresponding to the input CT images is generated. The probability image included in the probability image set 161b is an image in which a probability value is associated with each pixel of a corresponding CT image.

For example, in a case where the lesion identification models A1 and A2 are machine learning models using a neural network, the probability value described above is output from a last layer (output layer) of the neural network.

Each CT image including the organ region is also input to the image feature amount calculation unit 144. The image feature amount calculation unit 144 extracts a luminance value of each pixel of an organ region from an image region of each input CT image, and calculates an average value (average luminance) of the extracted luminance values as an image feature amount.

The detection result output unit 145 integrates the respective lesion identification results by the lesion identification models A1 and A2, and outputs a final detection result of the lesion region. At this time, for each pixel of the CT image as a processing target, the detection result output unit 145 integrates the probability value in the probability image in the probability image set 161a and the probability value in the probability image in the probability image set 161b at a ratio corresponding to the calculated image feature amount (average luminance). In this integration process, weighting addition using a weight coefficient corresponding to the image feature amount is performed on the probability value of each probability image.

By comparing the integrated probability value with a predetermined threshold value, the detection result output unit 145 outputs a final detection result indicating whether or not each pixel of the CT image is a lesion region. For example, a result image in which information indicating whether or not each pixel of the CT image as a processing target is a lesion region is associated with the pixel is generated. A result image set 171 illustrated in FIG. 7 includes result images respectively corresponding to the plurality of CT images as the processing targets.

According to the lesion detection process described above, the diagnosis support apparatus 100 may estimate the accumulation degree of fat in the CT image group based on the image feature amount calculated from the input CT image group. For example, it is estimated that the larger amount of fat is accumulated as the average luminance is lower. According to the accumulation degree of fat estimated based on the image feature amount, the diagnosis support apparatus 100 may appropriately integrate the lesion identification results by the lesion identification models A1 and A2 to obtain a final lesion identification result.

Accordingly, it is possible to accurately detect a lesion region from the simple CT image. For example, even in a case where a luminance difference between a lesion region and a normal region in an organ region is small, in a case where a magnitude relationship of luminances between the lesion region and the normal region is not uniform, and in a case where a luminance in the lesion region is not uniform, it is possible to improve detection accuracy of the lesion region.

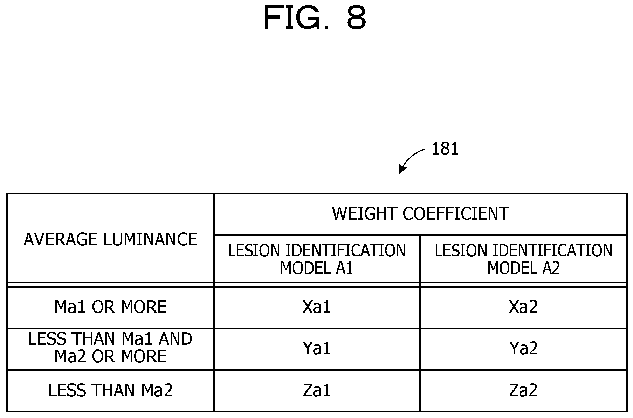

FIG. 8 is an example of a weight table indicating a correspondence relationship between an image feature amount and a weight coefficient. For example, the detection result output unit 145 may refer to a weight table 181 as illustrated in FIG. 8, and integrate each identification result of the lesion identification models A1 and A2.

As an example in FIG. 8, a region from which a calculated average luminance may be obtained is divided into three regions by using threshold values Ma1 and Ma2 (where Ma1 > Ma2), and a weight coefficient for each of the lesion identification models A1 and A2 is associated with the divided region. In a case where the average luminance is equal to or more than the threshold value Ma1, each of Xa1 and Xa2 is applied as the weight coefficient corresponding to the lesion identification models A1 and A2. In a case where the average luminance is less than the threshold value Ma1 and equal to or more than the threshold value Ma2, each of Ya1 and Ya2 is applied as the weight coefficient corresponding to the lesion identification models A1 and A2. In a case where the average luminance is less than the threshold value Ma2, each of Za1 and Za2 is applied as the weight coefficient corresponding to the lesion identification models A1 and A2.

Each of the weight coefficients Xa1, Xa2, Ya1, Ya2, Za1, and Za2 takes a value equal to or more than 0 and equal to or less than 1, and Xa1 + Xa2 = Ya1 + Ya2 = Za1 + Za2 = 1 is set. In a case where a weight coefficient corresponding to one of the lesion identification models A1 and A2 is 1, a weight coefficient corresponding to the other is 0. In this case, a probability value output from the one of the lesion identification models is used as it is.

It is estimated that the larger amount of fat is accumulated as the average luminance is lower. Therefore, as the average luminance is lower, the weight coefficient of the lesion identification model A2 corresponding to an abnormal finding is set to a higher value. For example, Xa1 > Ya1 > Za1 and Xa2 < Ya2 < Za2 are assumed. Xa1 > Xa2 and Za1 < Za2 are assumed. A magnitude relationship between Ya1 and Ya2 may be determined in accordance with setting values of the threshold values Ma1 and Ma2.

By using such a weight coefficient, the detection result output unit 145 may obtain a final lesion identification result by increasing a weight of a probability value by a lesion detection model in which a condition of a CT image used as learning data is close to a condition of an input CT image group among the lesion identification models A1 and A2. As a result, it is possible to accurately detect a lesion region from the simple CT image.

As the image feature amount, in addition to the average luminance of the organ region in the CT image group corresponding to one person as described above, for example, a ratio of high-luminance pixels having a luminance equal to or more than a predetermined threshold value in the organ region may be used. The ratio of such high-luminance pixels is calculated as a ratio of the number of high-luminance pixels included in the organ region to the total number of pixels of the organ region in the CT image group. In a case where such a ratio of high-luminance pixels is used as the image feature amount, a weight coefficient of the lesion identification model A2 corresponding to an abnormal finding may be set to a higher value as the ratio of high-luminance pixels is lower.

FIG. 6 illustrates an example in which the two lesion identification models A1 and A2 are generated by using two CT image sets having different findings. Meanwhile, as another example, three or more lesion identification models may be generated by using three or more CT image sets having different findings. For example, as a lesion identification model corresponding to an abnormal finding, a lesion identification model based on a CT image set with a low accumulation degree of fat and a lesion identification model based on a CT image set with a high accumulation degree of fat may be generated. In this case, a lesion identification model corresponding to a normal finding and the two lesion identification models corresponding to the abnormal finding are used at a time of lesion detection. As described above, in a case where three or more lesion identification models are used, at the time of lesion detection, lesion identification results by the three or more lesion identification models may be integrated at a ratio corresponding to the image feature amount.

As an abnormal event that may change the luminance relationship between the lesion region and the normal region, for example, calcification in the liver is also conceivable, in addition to the accumulation of fat as described above. A liver region in which calcification occurs appears bright over a CT image. Therefore, in a case where the calcification occurs, a luminance value of the entire liver region is increased. As a result, a luminance difference between the lesion region and the normal region is decreased, and it becomes difficult to identify a lesion region with high accuracy.

From this, for example, the diagnosis support apparatus 100 may generate a lesion identification model corresponding to a normal finding by using a CT image set in which calcification does not occur, and may generate a lesion identification model corresponding to an abnormal finding by using a CT image set in which calcification occurs. In this case, at a time of lesion identification, for example, the ratio of high-luminance pixels described above may be used as the image feature amount. For example, the lesion identification results (probability values) from the respective lesion identification models are integrated at a ratio corresponding to the ratio of high-luminance pixels in the input CT image set. At this time, as the ratio of high-luminance pixels is larger, a weight coefficient corresponding to the lesion identification model corresponding to the abnormal finding may be set to a higher value.

According to the above description, a plurality of lesion identification models are generated in consideration of the occurrence degree of one type of abnormal event that may change the luminance relationship between the lesion region and the normal region. Meanwhile, as another example, the plurality of lesion identification models may be generated in consideration of the degrees of occurrence of a plurality of types of abnormal events that may change the luminance relationship between the lesion region and the normal region.

For example, both accumulation of fat and calcification may be considered as the abnormal event. For example, a lesion identification model corresponding to a normal finding is generated by using a CT image set in which neither fat accumulation nor calcification does not appear. As lesion identification models corresponding to an abnormal finding, a lesion identification model based on a CT image set in which fat accumulation appears and a lesion identification model based on a CT image set in which calcification appears are generated. At a time of detecting a lesion, the lesion identification results by the respective lesion identification models may be integrated at a ratio corresponding to the image feature amount.

Next, the process of the diagnosis support apparatus 100 according to the second embodiment will be described with reference to a flowchart.

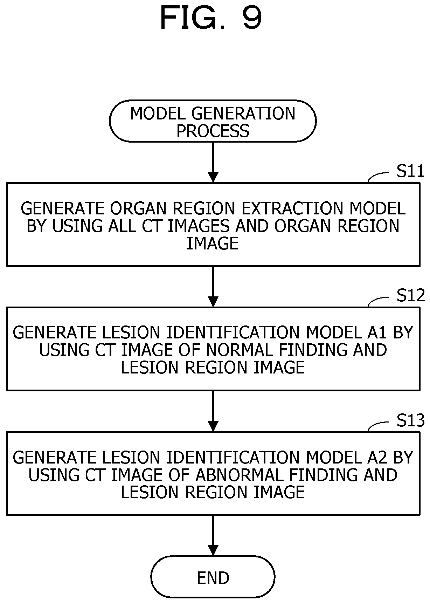

First, a procedure of a model generation process for generating the above lesion identification models A1 and A2 will be described with reference to FIG. 9. FIG. 9 is an example of a flowchart illustrating the procedure of the model generation process.

[STEP S11] The learning processing unit 131 generates an organ region extraction model by performing machine learning using all the CT images 112a stored in the learning data storage unit 110 and the organ region image 114a corresponding to each CT image 112a. In this machine learning, information on each pixel in the organ region image 114a is used as correct answer data. The learning processing unit 131 stores the model parameter 121 indicating the generated organ region extraction model in the model parameter storage unit 120.

As an example in the present embodiment, the organ region extraction model is generated by using the same learning data as those used when the lesion identification models A1 and A2 are generated. Meanwhile, an organ region extraction model generated in advance by using different learning data may be used as the organ region extraction model.

[STEP S12] Based on the finding information 115, the learning processing unit 132 extracts the learning data set 111 of a normal finding from the learning data set 111 stored in the learning data storage unit 110. The learning processing unit 132 extracts the CT image 112a including an organ region based on the organ region image 114a from the extracted learning data set 111.

By performing machine learning using the extracted CT images 112a and the lesion region images 113a corresponding to these CT images 112a, the learning processing unit 132 generates the lesion identification model A1 corresponding to the normal finding. In this machine learning, information on each pixel in the lesion region image 113a is used as correct answer data. The learning processing unit 131 stores the model parameter 122a indicating the generated lesion identification model A1 in the model parameter storage unit 120.

[STEP S13] Based on the finding information 115, the learning processing unit 132 extracts the learning data set 111 of an abnormal finding, from the learning data sets 111 stored in the learning data storage unit 110. The learning processing unit 132 extracts the CT image 112a including an organ region based on the organ region image 114a from the extracted learning data set 111.

By performing machine learning using the extracted CT images 112a and the lesion region images 113a corresponding to these CT images 112a, the learning processing unit 132 generates the lesion identification model A2 corresponding to the abnormal finding. In this machine learning, information on each pixel in the lesion region image 113a is used as correct answer data. The learning processing unit 131 stores the model parameter 122b indicating the generated lesion identification model A2 in the model parameter storage unit 120.

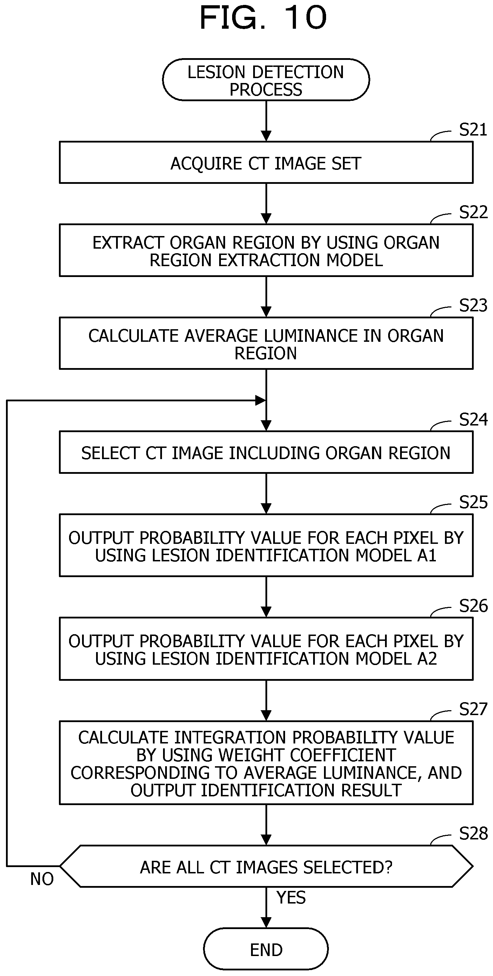

Next, a procedure of a lesion detection process of detecting a lesion region by using the generated lesion identification models A1 and A2 will be described with reference to FIG. 10. FIG. 10 is an example of a flowchart illustrating the procedure of the lesion detection process.

[STEP S21] The image acquisition unit 141 acquires a CT image set obtained by imaging for a diagnosis target person from the CT apparatus 50.

[STEP S22] The organ region extraction unit 142 inputs each CT image included in the CT image set to an organ region extraction model based on the model parameter 121. Thus, the organ region extraction unit 142 identifies whether or not each pixel in each CT image is an organ region. For example, an organ region image in which information indicating whether or not each pixel of the CT image is an organ region is associated with the pixel is generated.

The organ region extraction unit 142 extracts a CT image including an organ region (a CT image including one or more pixels of the organ region) from the CT image set. In and after the next step S23, the process is executed by using the extracted CT image.

[STEP S23] The image feature amount calculation unit 144 acquires a pixel value of each pixel of the organ region from all the CT images including the organ region, and converts the acquired pixel value into a luminance value. The image feature amount calculation unit 144 calculates an average value of the luminance values after the conversion, for example, an average luminance in the organ region.

[STEP S24] The lesion identification unit 143 selects a CT image as a processing target from the CT images including the organ region.

[STEP S25] The lesion identification unit 143 inputs the CT image as the processing target to the lesion identification model A1 based on the model parameter 122a. Thus, the lesion identification unit 143 outputs a probability value indicating a lesion region for each pixel of the CT image.

[STEP S26] The lesion identification unit 143 inputs the CT image as the processing target to the lesion identification model A2 based on the model parameter 122b. Thus, the lesion identification unit 143 outputs a probability value indicating a lesion region for each pixel of the CT image.

[STEP S27] The detection result output unit 145 acquires a weight coefficient corresponding to the average luminance calculated in step S23 from the weight table 181. Thus, the weight coefficients respectively corresponding to the lesion identification models A1 and A2 are acquired. The detection result output unit 145 multiplies the probability values output in steps S25 and S26 by the weight coefficients corresponding to the lesion identification models A1 and A2, respectively, and adds each of the obtained multiplication results to calculate an integration probability value for each pixel. The detection result output unit 145 compares the calculated integration probability value with a predetermined threshold value, identifies a pixel having an integration probability value equal to or more than the threshold value as a lesion region, and identifies a pixel having an integration probability value less than the threshold value as a normal region. For example, the detection result output unit 145 generates and outputs a result image in which information indicating whether or not each pixel of the CT image is a lesion region is associated with the pixel.

[STEP S28] The lesion identification unit 143 determines whether all the CT images including the organ region are selected. In a case where there is an unselected CT image, the process proceeds to step S24, and one unselected CT image is selected. On the other hand, in a case where all the corresponding CT images are selected, the lesion detection process is ended.

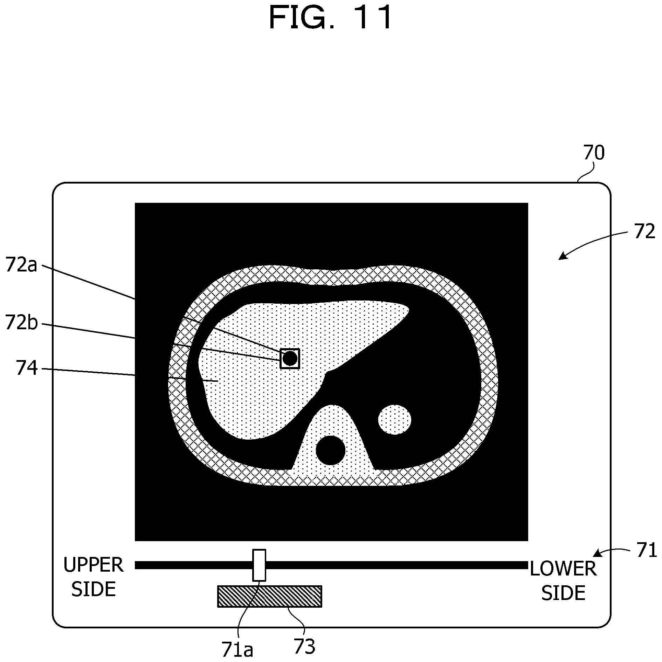

FIG. 11 is a diagram illustrating a screen display example of a detection result of a lesion region. By using a result image generated for each CT image including an organ region, the detection result output unit 145 may output information indicating a detection result of a lesion region. For example, the detection result output unit 145 may display a result display screen 70 as illustrated in FIG. 11 on the display device 104a. The result display screen 70 includes a slice selection unit 71, a CT image display unit 72, and a lesion detection region display unit 73.

The slice selection unit 71 may select a slice plane of a CT image to be displayed on the CT image display unit 72 by moving a handle 71a over a slider. A CT image corresponding to the slice plane selected by the slice selection unit 71 is displayed on the CT image display unit 72. A lesion region is displayed in the CT image based on a result image corresponding to the same slice plane. As an example in FIG. 11, a rectangular 72b surrounding a lesion region 72a is superimposed and displayed in the CT image. As another display method, the lesion region 72a may be displayed in a specific color. The lesion region 72a is a region of pixels associated with information indicating the lesion region in the result image. The organ region 74 may be displayed in another specific color.

The lesion detection region display unit 73 indicates a range of a slice plane in which a lesion region is detected (a slice plane corresponding to a result image in which one or more pixels of information indicating the lesion region are included) in a movable region of the handle 71a in the slice selection unit 71. By the display on the lesion detection region display unit 73, a user may easily recognize the slice plane in which the lesion region is detected, quickly display a tomographic image corresponding to the slice plane, and check the lesion region over the tomographic image.

### [Third Embodiment]

Next, description is given of a third embodiment in which an example in which a part of the process of the diagnosis support apparatus 100 according to the second embodiment is modified. According to the third embodiment, the following points are different from those of the second embodiment. According to the third embodiment, a CT image set for learning is classified into a plurality of CT image groups in accordance with a range of an image feature amount calculated from the CT image set. A plurality of lesion identification models are generated by using each of the classified CT image groups as learning data.

As an example in the following description, it is assumed that a CT image set for learning is classified into two CT image groups and two lesion identification models are generated.

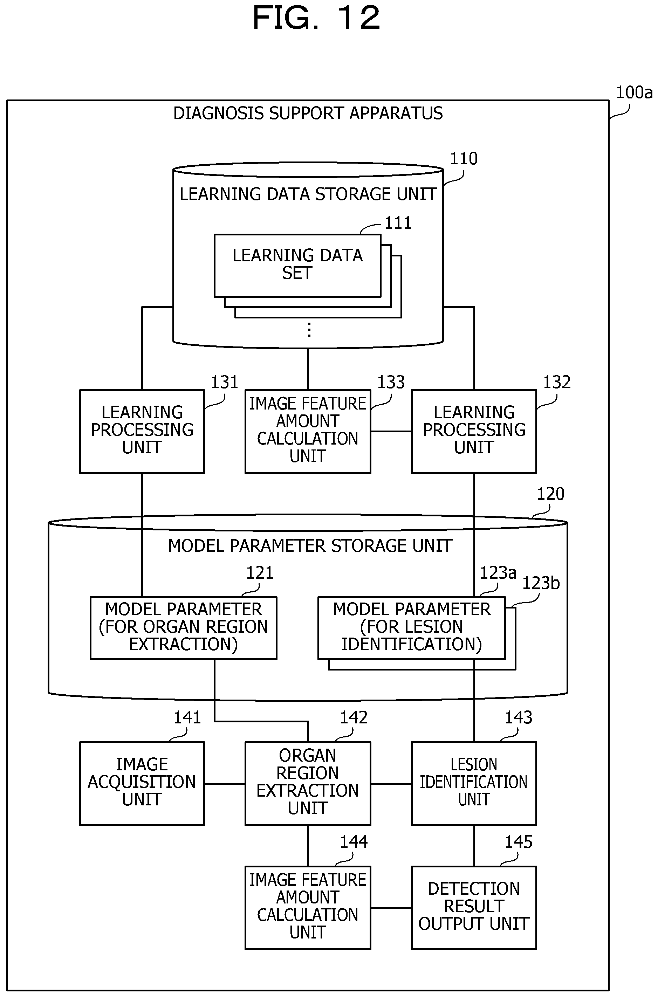

FIG. 12 is a diagram illustrating a configuration example of a processing function provided by a diagnosis support apparatus according to the third embodiment. In FIG. 12, components in the same manner as the components in FIG. 4 are denoted by the same reference numerals.

A diagnosis support apparatus 100a according to the third embodiment further includes an image feature amount calculation unit 133, in addition to the learning processing units 131 and 132, the image acquisition unit 141, the organ region extraction unit 142, the lesion identification unit 143, the image feature amount calculation unit 144, and the detection result output unit 145 illustrated in FIG. 4. A process of the image feature amount calculation unit 133 is realized by a processor included in the diagnosis support apparatus 100a executing a predetermined program. From each in the CT image set 112 stored in the learning data storage unit 110, the image feature amount calculation unit 133 calculates an image feature amount of the same type as the image feature amount calculated by the image feature amount calculation unit 144.

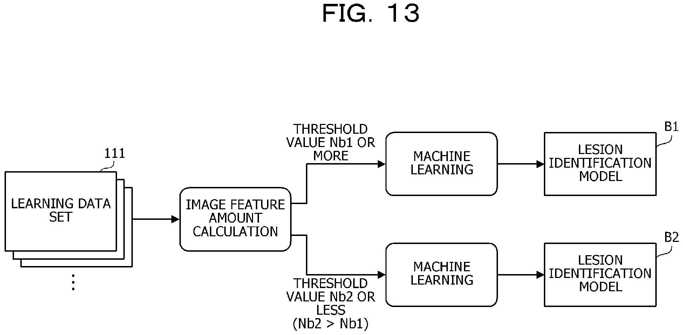

The CT image set 112 stored in the learning data storage unit 110 is classified into a CT image group of which a calculated image feature amount is included in a first range and a CT image group of which a calculated image feature amount is included in a second range. The learning processing unit 132 generates a lesion identification model B1 (see FIG. 13) by machine learning using CT images included in one CT image group as learning data, and stores a model parameter 123a indicating the generated lesion identification model B1 in the model parameter storage unit 120. The learning processing unit 132 generates a lesion identification model B2 (see FIG. 13) by machine learning using CT images included in the other CT image group as learning data, and stores a model parameter 123b indicating the generated lesion identification model B2 in the model parameter storage unit 120.

Based on the model parameters 123a and 123b, the lesion identification unit 143 calculates a probability value of a lesion region for each pixel by using the lesion identification models B1 and B2. The detection result output unit 145 outputs a final lesion detection result by integrating the calculated probability values at a ratio corresponding to the image feature amount calculated by the image feature amount calculation unit 144.

FIG. 13 is a diagram for describing a generation process of a lesion identification model.

From each in the CT image set 112 stored in the learning data storage unit 110, the image feature amount calculation unit 133 calculates an image feature amount of the same type as the image feature amount calculated by the image feature amount calculation unit 144. Here, it is assumed that an average luminance in an organ region is calculated as the image feature amount.

The learning processing unit 132 classifies the CT image set 112 stored in the learning data storage unit 110 into a first CT image group of which an image feature amount is included in a first range and a second CT image group of which an image feature amount is included in a second range. The learning processing unit 132 generates the lesion identification model B1 by machine learning using the first CT image group as learning data, and generates the lesion identification model B2 by machine learning using the second CT image group as learning data.

The first range for the image feature amount is a range equal to or more than a threshold value Nb1, and the second range is a range equal to or less than a threshold value Nb2. In this case, substantially, the first CT image group corresponds to a CT image group of a normal finding in the second embodiment, and the second CT image group corresponds to a CT image group of an abnormal finding in the second embodiment. Accordingly, substantially, the lesion identification model B1 corresponds to the normal finding, and the lesion identification model B2 corresponds to the abnormal finding.

The first range and the second range may be divided by one threshold value. Meanwhile, in the present embodiment, the first and second ranges are set by using the two threshold values Nb1 and Nb2, and Nb2 > Nb1 is set. Thus, the threshold values Nb1 and Nb2 are set such that a part of a lower limit side in the first range and a part of an upper limit side in the second range overlap with each other.

A range of an image feature amount (average luminance) that may be calculated from the CT image set of the normal finding and a range of an image feature amount (average luminance) that may be calculated from the CT image set of the abnormal finding are not necessarily divided with a specific threshold value as a boundary. In a case where the image feature calculated from the CT image set has a value near the boundary of these ranges, it is not necessarily clear whether the CT image set is a normal finding or an abnormal finding. By overlapping the part of the first range and the part of the second range by setting Nb2 > Nb1, it is possible to generate the lesion identification models B1 and B2 in consideration of such uncertainty. For example, even in a case where the feature amount of the CT image set input at a time of lesion detection is a value near an overlapping region of the first and second ranges, it is possible to improve accuracy of the lesion identification by using the lesion identification models B1 and B2.

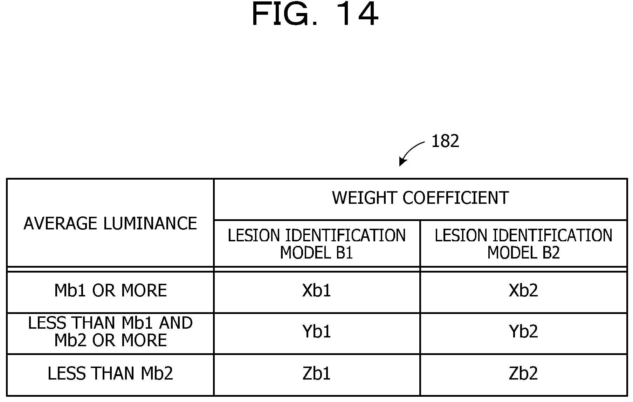

At a time of detecting a lesion region, the lesion identification models A1 and A2 may be used instead of the lesion identification models B1 and B2 in FIG. 7, respectively, and a final lesion detection result may be output in the same procedure as in FIG. 7. A weight coefficient as illustrated in FIG. 14 may be used in the integration process of the probability value from the lesion identification model B1 and the probability value from the lesion identification model B2.

FIG. 14 is an example of a weight table indicating a correspondence relationship between an image feature amount and a weight coefficient. For example, the detection result output unit 145 may refer to a weight table 182 as illustrated in FIG. 14, and integrate each identification result of the lesion identification models B1 and B2.

As an example in FIG. 14, a region from which a calculated average luminance may be obtained is divided into three regions by using threshold values Mb1 and Mb2 (where Mb1 > Mb2), and a weight coefficient for each of the lesion identification models B1 and B2 are associated with the divided region. In a case where the average luminance is equal to or more than the threshold value Mb1, each of Xb1 and Xb2 is applied as the weight coefficient corresponding to the lesion identification models B1 and B2. In a case where the average luminance is less than the threshold value Mb1 and equal to or more than the threshold value Mb2, each of Yb1 and Yb2 is applied as the weight coefficient corresponding to the lesion identification models B1 and B2. In a case where the average luminance is less than the threshold value Mb2, each of Zb1 and Zb2 is applied as the weight coefficient corresponding to the lesion identification models B1 and B2.

Each of the weight coefficients Xb1, Xb2, Yb1, Yb2, Zb1, and Zb2 takes a value equal to or more than 0 and equal to or less than 1, and Xb1 + Xb2 = Yb1 + Yb2 = Zb1 + Zb2 = 1 is set. In a case where a weight coefficient corresponding to one of the lesion identification models B1 and B2 is 1, a weight coefficient corresponding to the other is 0. In this case, a probability value output from the one of the lesion identification models is used as it is.

It is estimated that the larger amount of fat is accumulated as the average luminance is lower. Therefore, as the average luminance is lower, the weight coefficient of the lesion identification model B2 corresponding to an abnormal finding is set to a higher value. For example, Xb1 > Yb1 > Zb1 and Xb2 < Yb2 < Zb2 are assumed. Xb1 > Xb2 and Zb1 < Zb2 are assumed. A magnitude relationship between Yb1 and Yb2 may be determined in accordance with setting values of the threshold values Mb1 and Mb2.

By using such a weight coefficient, the detection result output unit 145 may obtain a final lesion identification result by increasing a weight of a probability value by a lesion detection model in which a condition of a CT image used as learning data is close to a condition of an input CT image group among the lesion identification models B1 and B2. As a result, it is possible to accurately detect a lesion region from the simple CT image.

As the image feature amount calculated by the image feature amount calculation units 133 and 144, in addition to the average luminance of the organ region in the CT image group corresponding to one person as described above, for example, a ratio of high-luminance pixels having a luminance equal to or more than a predetermined threshold value in the organ region may be used. In a case where such a ratio of high-luminance pixels is used as the image feature amount, the lesion identification model B1 is generated by using a CT image set in which the ratio of high-luminance pixels is equal to or more than the threshold value Nb1a at a time of learning. The lesion identification model B2 is generated by using a CT image set in which the ratio of high-luminance pixels is equal to or less than the threshold value Nb2a (> Nb1a). At a time of lesion detection, the weight coefficient of the lesion identification model B2 may be set to a higher value as the ratio of high-luminance pixels is lower.

FIG. 13 illustrates an example in which the two lesion identification models B1 and B2 are generated by using two CT image sets having different ranges of image feature amounts. Meanwhile, as another example, three or more lesion identification models may be generated by using three or more CT image sets having different ranges of image feature amounts. In this case, at a time of lesion detection, lesion identification results by the three or more lesion identification models may be integrated at a ratio corresponding to the image feature amount.

A plurality of types of image feature amounts may be used. For example, the image feature amount calculation units 133 and 144 calculate the average luminance and the ratio of high-luminance pixels described above. At a time of learning, for example, Nbx is used as a threshold value of the average luminance, and Nby is used as a threshold value of the ratio of high-luminance pixels. In this case, the CT image set 112 for learning is classified into, for example, a CT image group having an average luminance ≥ Nbx and a ratio of high-luminance pixels ≥ Nby, a CT image group having an average luminance ≥ Nbx and a ratio of high-luminance pixels < Nby, a CT image group having an average luminance < Nbx and a ratio of high-luminance pixels ≥ Nby, and a CT image group having an average luminance < Nbx and a ratio of high-luminance pixels < Nby. An individual lesion identification model is generated by using each of the classified CT image groups. At a time of lesion detection, the lesion identification results by the respective lesion identification models may be integrated at a ratio corresponding to a combination of the average luminance and the ratio of high-luminance pixels based on the input CT image set.

In the same manner as the second embodiment, calcification in a liver may be considered as an abnormal event that may change a luminance relationship between a lesion region and a normal region. In this case, for example, the image feature amount calculation units 133 and 144 calculate the ratio of high-luminance pixels described above. At a time of learning, the lesion identification model B1 is generated by using a CT image set in which the ratio of high-luminance pixels is equal to or less than the threshold value Nb2b. The lesion identification model B2 is generated by using a CT image set in which the ratio of high-luminance pixels is equal to or more than the threshold value Nb1b (< Nb2a). At a time of lesion detection, the weight coefficient of the lesion identification model B2 may be set to a higher value as the ratio of high-luminance pixels is higher.

Next, the process of the diagnosis support apparatus 100a according to the third embodiment will be described with reference to a flowchart.

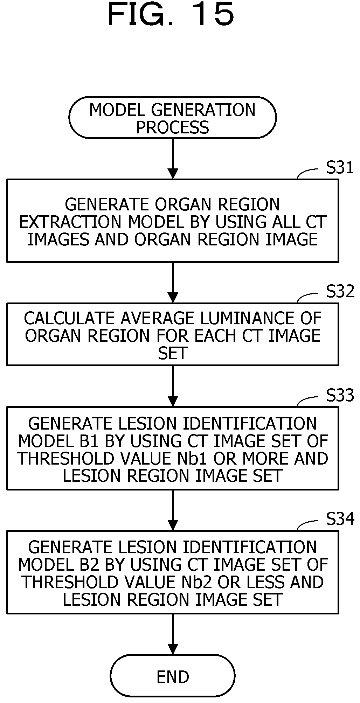

First, a procedure of a model generation process for generating the above lesion identification models B1 and B2 will be described with reference to FIG. 15. FIG. 15 is an example of a flowchart illustrating the procedure of the model generation process.

[STEP S31] The learning processing unit 131 generates an organ region extraction model by performing machine learning using all the CT images 112a stored in the learning data storage unit 110 and the organ region image 114a corresponding to each CT image 112a. In this machine learning, information on each pixel in the organ region image 114a is used as correct answer data. The learning processing unit 131 stores the model parameter 121 indicating the generated organ region extraction model in the model parameter storage unit 120.

[STEP S32] For each in the CT image set 112 stored in the learning data storage unit 110, the image feature amount calculation unit 133 calculates an average luminance in an organ region.

[STEP S33] From the CT image set 112 stored in the learning data storage unit 110, the learning processing unit 132 extracts the CT image set 112 of which the calculated average luminance is equal to or more than the threshold value Nb1. Based on the organ region image 114a, the learning processing unit 132 extracts the CT image 112a including the organ region from the extracted CT image set 112.

By performing machine learning using the extracted CT images 112a and the lesion region images 113a corresponding to these CT images 112a, the learning processing unit 132 generates the lesion identification model B1 corresponding to a normal finding. In this machine learning, information on each pixel in the lesion region image 113a is used as correct answer data. The learning processing unit 131 stores the model parameter 123a indicating the generated lesion identification model B1 in the model parameter storage unit 120.

[STEP S34] From the CT image set 112 stored in the learning data storage unit 110, the learning processing unit 132 extracts the CT image set 112 of which the calculated average luminance is equal to or less than the threshold value Nb2. Based on the organ region image 114a, the learning processing unit 132 extracts the CT image 112a including the organ region from the extracted CT image set 112.

By performing machine learning using the extracted CT images 112a and the lesion region images 113a corresponding to these CT images 112a, the learning processing unit 132 generates the lesion identification model B2 corresponding to an abnormal finding. In this machine learning, information on each pixel in the lesion region image 113a is used as correct answer data. The learning processing unit 131 stores the model parameter 123b indicating the generated lesion identification model B2 in the model parameter storage unit 120.

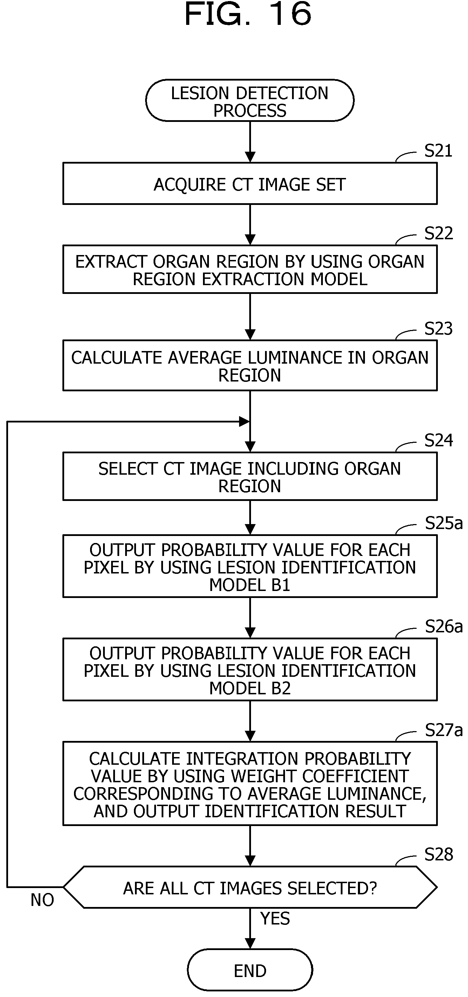

Next, a procedure of a lesion detection process of detecting a lesion region by using the generated lesion identification models B1 and B2 will be described with reference to FIG. 16. FIG. 16 is an example of a flowchart illustrating the procedure of the lesion detection process.

In FIG. 16, processing steps having the same processing contents as the processing contents illustrated in FIG. 10 are denoted by the same step numbers. In FIG. 16, steps S25a to S27a are executed instead of steps S25 to S27 in FIG. 10.

[STEP S25a] The lesion identification unit 143 inputs a CT image as a processing target to the lesion identification model B1 based on the model parameter 123a. Thus, the lesion identification unit 143 outputs a probability value indicating a lesion region for each pixel of the CT image.

[STEP S26a] The lesion identification unit 143 inputs the CT image as the processing target to the lesion identification model B2 based on the model parameter 123b. Thus, the lesion identification unit 143 outputs a probability value indicating a lesion region for each pixel of the CT image.

[STEP S27a] The detection result output unit 145 acquires a weight coefficient corresponding to the average luminance calculated in step S23 from the weight table 182. Thus, the weight coefficients respectively corresponding to the lesion identification models B1 and B2 are acquired. The detection result output unit 145 multiplies the probability values output in steps S25a and S26a by the weight coefficients corresponding to the lesion identification models B1 and B2, respectively, and adds each of the obtained multiplication results to calculate an integration probability value for each pixel. The detection result output unit 145 compares the calculated integration probability value with a predetermined threshold value, identifies a pixel having an integration probability value equal to or more than the threshold value as a lesion region, and identifies a pixel having an integration probability value less than the threshold value as a normal region. For example, the detection result output unit 145 generates and outputs a result image in which information indicating whether or not each pixel of the CT image is a lesion region is associated with the pixel.

### [Fourth Embodiment]

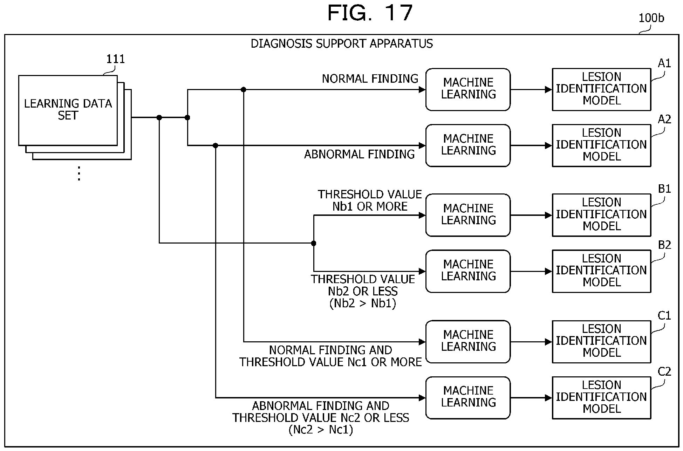

All of the lesion identification models A1 and A2 generated in the second embodiment and the lesion identification models B1 and B2 generated in the third embodiment may be used to execute the lesion detection process. Lesion identification models C1 and C2 (see FIG. 17) generated in consideration of both a difference in finding and a difference in image feature amount may be used. An example of a diagnosis support apparatus that executes a lesion detection process by using these lesion identification models A1, A2, B1, B2, C1, and C2 will be described in a fourth embodiment below.

FIG. 17 is a diagram for describing a model generation process of the diagnosis support apparatus according to the fourth embodiment. A diagnosis support apparatus 100b according to the fourth embodiment includes the learning processing units 131 and 132, the image feature amount calculation unit 133, the image acquisition unit 141, the organ region extraction unit 142, the lesion identification unit 143, the image feature amount calculation unit 144, and the detection result output unit 145 in FIG. 12.

By the same procedure as the procedure in the second embodiment, the learning processing unit 132 classifies the CT image set 112 included in the learning data set 111 into the CT image set 112 of a normal finding and the CT image set 112 of an abnormal finding. The learning processing unit 132 generates the lesion identification model A1 by machine learning using the CT image set 112 of the normal finding, and generates the lesion identification model A2 by machine learning using the CT image set 112 of the abnormal finding.

According to the same procedure as the procedure in the third embodiment, the learning processing unit 132 classifies the CT image set 112 included in the learning data set 111 into a first CT image group having an image feature amount equal to or more than the threshold value Nb1 and a second CT image group having an image feature amount equal to or less than the threshold value Nb2 (> Nb1). The learning processing unit 132 generates the lesion identification model B1 by machine learning using the first CT image group, and generates the lesion identification model B2 by machine learning using the second CT image group.

The learning processing unit 132 extracts a third CT image group of the CT image set 112 in which an image feature amount is equal to or more than the threshold value Nc1, from the CT image set 112 of the normal finding. The learning processing unit 132 extracts a fourth CT image group of the CT image set 112 in which an image feature amount is equal to or less than the threshold value Nc2, from the CT image set 112 of the abnormal finding. Here, Nc2 > Nc1 is set. The learning processing unit 132 generates the lesion identification model C1 by machine learning using the third CT image group, and generates the lesion identification model C2 by machine learning using the fourth CT image group.

By using the lesion identification models A1, A2, B1, B2, C1, and C2, the lesion identification unit 143 calculates a probability value indicating a lesion region for each pixel. The detection result output unit 145 outputs a final lesion detection result by integrating the calculated probability values at a ratio corresponding to the image feature amount calculated by the image feature amount calculation unit 144.

There may be a special case where a relationship between a finding and an image feature does not coincide with a theoretical relationship, such as a case where fat is accumulated but an organ region is imaged relatively brightly in a real CT image. The lesion identification models C1 and C2 are trained models for enabling accurate detection of a lesion region even in a case where such a special case occurs. For example, by setting the threshold value Nc1 to a relatively low value, it is possible to identify the lesion region with high accuracy even in a case where a CT image set having a finding that fat is not accumulated but having a relatively low average luminance is input to the lesion identification model C1. By setting the threshold value Nc2 to a relatively high value, it is possible to identify the lesion region with high accuracy even in a case where a CT image set having a relatively high average luminance but having a finding that fat is accumulated is input to the lesion identification model C2.

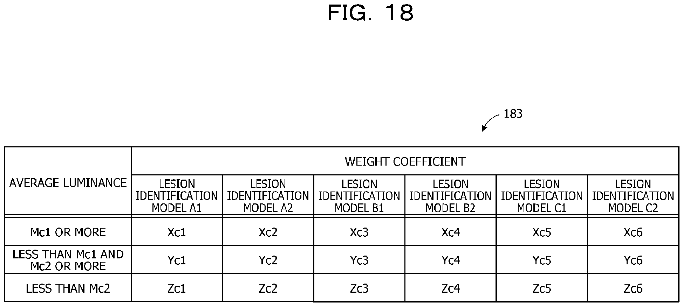

FIG. 18 is an example of a weight table indicating a correspondence relationship between an image feature amount and a weight coefficient. For example, the detection result output unit 145 may refer to a weight table 183 as illustrated in FIG. 18, and integrate each identification result of the lesion identification models A1, A2, B1, B2, C1, and C2.

As an example in FIG. 18, a region from which a calculated average luminance may be obtained is divided into three regions by using threshold values Mc1 and Mc2 (where Mc1 > Mc2). A weight coefficient of each of the lesion identification models A1, A2, B1, B2, C1, and C2 is associated for each of the divided regions.

In a case where the average luminance is equal to or more than the threshold value Mc1, Xc1 to Xc6 are applied as the weight coefficients corresponding to the lesion identification models A1, A2, B1, B2, C1, and C2, respectively. In a case where the average luminance is less than the threshold value Mc1 and equal to or more than the threshold value Mc2, Yc1 to Yc6 are applied as the weight coefficients corresponding to the lesion identification models A1, A2, B1, B2, C1, and C2, respectively. In a case where the average luminance is less than the threshold value Mc2, the weight coefficients Zc1 to Zc6 are applied as the weight coefficients corresponding to the lesion identification models A1, A2, B1, B2, C1, and C2, respectively.

Each of the weight coefficients Xc1 to Xc6, Yc1 to Yc6, and Zc1 to Zc6 takes a value equal to or more than 0 and equal to or less than 1. Xc1 + Xc2 + Xc3 + Xc4 + Xc5 + Xc6 = Yc1 + Yc2 + Yc3 + Yc4 + Yc5 + Yc6 = Zc1 + Zc2 + Zc3 + Zc4 + Zc5 + Zc6 = 1 is set.

As the average luminance is lower, the weight coefficient of the lesion identification model A2 among the lesion identification models A1 and A2 is set to a higher value, and the weight coefficient of the lesion identification model B2 among the lesion identification models B1 and B2 is set to a higher value. For example, Xc1 > Yc1 > Zc1, Xc2 < Yc2 < Zc2, Xc3 > Yc3 > Zc3, and Xc4 < Yc4 < Zc4 are assumed. Xc1 > Xc2, Zc1 < Zc2, Xc3 > Xc4, and Zc3 < Zc4 are assumed. A magnitude relationship between Yc1 and Yc2 and a magnitude relationship between Yc3 and Yc4 may be determined in accordance with setting values of the threshold values Mc1 and Mc2.

On the other hand, the lesion identification models C1 and C2 are generated by machine learning excluding learning data (learning data of outliers) in special cases such as a case where an image has a normal finding but is dark or a case where fat is accumulated but the image is light. By such machine learning, a lesion identification model with a more robust normal finding or abnormal finding is generated. As for the weight coefficients, Xc5 > Yc5 > Zc5, Xc6 < Yc6 < Zc6, Xc5 > Xc6, and Zc5 < Zc6 are assumed. A magnitude relationship between Yc5 and Yc6 may be determined in accordance with setting values of the threshold values Mc1 and Mc2.

By using such a weight coefficient, the detection result output unit 145 may obtain a final lesion identification result by increasing a weight of a probability value by a lesion detection model in which a condition of a CT image used as learning data is close to a condition of an input CT image group among the lesion identification models A1, A2, B1, B2, C1, and C2. As a result, it is possible to accurately detect a lesion region from the simple CT image.

Next, the process of the diagnosis support apparatus 100b according to the fourth embodiment will be described with reference to a flowchart.

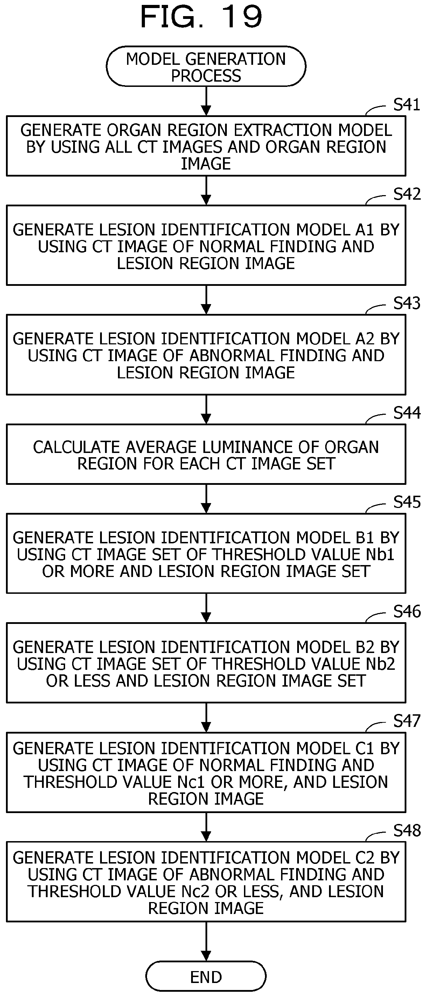

First, a procedure of a model generation process for generating the above lesion identification models A1, A2, B1, B2, C1, and C2 will be described with reference to FIG. 19. FIG. 19 is an example of a flowchart illustrating the procedure of the model generation process.

[STEP S41] With the same procedure as the procedure in step S11 in FIG. 9 and step S31 in FIG. 15, the learning processing unit 131 generates an organ region extraction model, and stores the model parameter 121 indicating the generated organ region extraction model in the model parameter storage unit 120.

[STEP S42] With the same procedure as the procedure in step S12 in FIG. 9, the learning processing unit 131 generates the lesion identification model A1, and stores the model parameter 122a indicating the generated lesion identification model A1 in the model parameter storage unit 120.

[STEP S43] With the same procedure as the procedure in step S13 in FIG. 9, the learning processing unit 131 generates the lesion identification model A2, and stores the model parameter 122b indicating the generated lesion identification model A2 in the model parameter storage unit 120.

[STEP S44] With the same procedure as the procedure in step S32 in FIG. 15, the image feature amount calculation unit 133 calculates an average luminance in an organ region, for each in the CT image set 112 stored in the learning data storage unit 110.

[STEP S45] With the same procedure as the procedure in step S33 in FIG. 15, the learning processing unit 132 generates the lesion identification model B1, and stores the model parameter 123a indicating the generated lesion identification model B1 in the model parameter storage unit 120.

[STEP S46] With the same procedure as the procedure in step S34 in FIG. 15, the learning processing unit 132 generates the lesion identification model B2, and stores the model parameter 123b indicating the generated lesion identification model B2 in the model parameter storage unit 120.

[STEP S47] From among the CT images 112a of a normal finding used as learning data in step S42, the learning processing unit 132 extracts the CT image 112a in which an average luminance calculated from the CT image set corresponding in step S44 is equal to or more than the threshold value Nc1. By performing machine learning using the extracted CT images 112a and the lesion region images 113a corresponding to these CT images 112a, the learning processing unit 132 generates the lesion identification model C1. In this machine learning, information on each pixel in the lesion region image 113a is used as correct answer data. The learning processing unit 131 stores a model parameter indicating the generated lesion identification model C1 in the model parameter storage unit 120.

[STEP S48] From among the CT images 112a of an abnormal finding used as learning data in step S43, the learning processing unit 132 extracts the CT image 112a in which the average luminance calculated from the CT image set corresponding in step S44 is equal to or less than the threshold value Nc2. By performing machine learning using the extracted CT images 112a and the lesion region images 113a corresponding to these CT images 112a, the learning processing unit 132 generates the lesion identification model C2. In this machine learning, information on each pixel in the lesion region image 113a is used as correct answer data. The learning processing unit 131 stores a model parameter indicating the generated lesion identification model C2 in the model parameter storage unit 120.

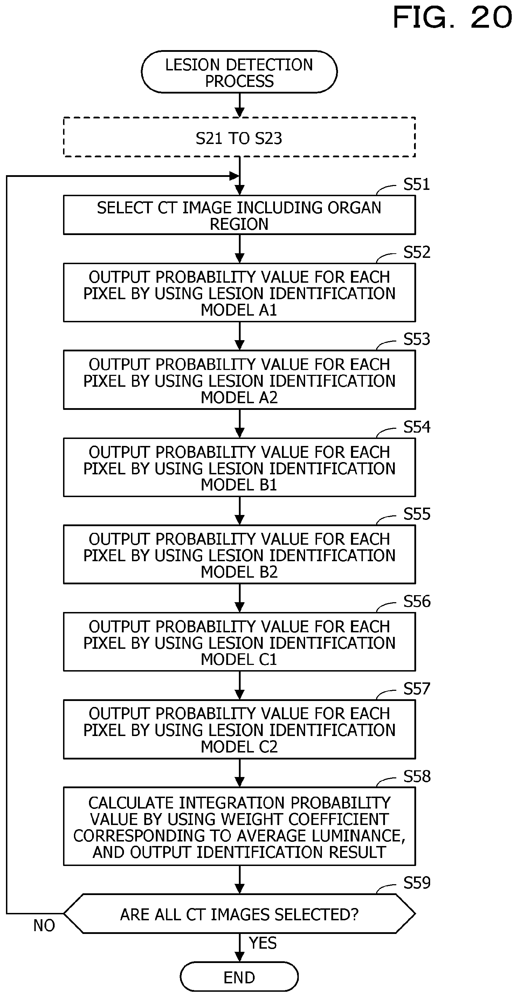

Next, a procedure of a lesion detection process of detecting a lesion region by using the generated lesion identification models A1, A2, B1, B2, C1, and C2 will be described with reference to FIG. 20. FIG. 20 is an example of a flowchart illustrating a procedure of a lesion detection process.

First, in the processes in FIG. 20, the processes in steps S21 to S23 in FIG. 10 are executed. Thus, an average luminance in an organ region of a CT image set is calculated. After that, the process in and after the step S51 is executed.

[STEP S51] The lesion identification unit 143 selects a CT image as a processing target from the CT images including the organ region.

[STEP S52] The lesion identification unit 143 inputs the CT image as the processing target to the lesion identification model A1 based on the model parameter 122a. Thus, the lesion identification unit 143 outputs a probability value indicating a lesion region for each pixel of the CT image.

[STEP S53] The lesion identification unit 143 inputs the CT image as the processing target to the lesion identification model A2 based on the model parameter 122b. Thus, the lesion identification unit 143 outputs a probability value indicating a lesion region for each pixel of the CT image.

[STEP S54] The lesion identification unit 143 inputs a CT image as a processing target to the lesion identification model B1 based on the model parameter 123a. Thus, the lesion identification unit 143 outputs a probability value indicating a lesion region for each pixel of the CT image.

[STEP S55] The lesion identification unit 143 inputs the CT image as the processing target to the lesion identification model B2 based on the model parameter 123b. Thus, the lesion identification unit 143 outputs a probability value indicating a lesion region for each pixel of the CT image.

[STEP S56] The lesion identification unit 143 inputs the CT image as the processing target to the lesion identification model C1, and outputs a probability value indicating a lesion region for each pixel of the CT image.

[STEP S57] The lesion identification unit 143 inputs the CT image as the processing target to the lesion identification model C2, and outputs a probability value indicating a lesion region for each pixel of the CT image.

[STEP S58] The detection result output unit 145 acquires a weight coefficient corresponding to the average luminance calculated in step S23 from the weight table 183. Thus, the weight coefficients respectively corresponding to the lesion identification models A1, A2, B1, B2, C1, and C2 are acquired. The detection result output unit 145 multiplies the probability values output in step S52 to S57 by the weight coefficients corresponding to the lesion identification models A1, A2, B1, B2, C1, and C2, respectively, and adds each of the obtained multiplication results to calculate an integration probability value for each pixel. The detection result output unit 145 compares the calculated integration probability value with a predetermined threshold value, identifies a pixel having an integration probability value equal to or more than the threshold value as a lesion region, and identifies a pixel having an integration probability value less than the threshold value as a normal region. For example, the detection result output unit 145 generates and outputs a result image in which information indicating whether or not each pixel of the CT image is a lesion region is associated with the pixel.

[STEP S59] The lesion identification unit 143 determines whether all the CT images including the organ region are selected. In a case where there is an unselected CT image, the process proceeds to step S51, and one unselected CT image is selected. On the other hand, in a case where all the corresponding CT images are selected, the lesion detection process is ended.

For example, the diagnosis support apparatus may execute the lesion detection process by combining two sets of a set of the lesion identification models A1 and A2, a set of the lesion identification models B1 and B2, and a set of the lesion identification models C1 and C2.

For example, the lesion detection process is executed by using the lesion identification models A1 and A2 and the lesion identification models C1 and C2. It may be said that the lesion identification models C1 and C2 cover a special case where a lesion region may not be identified with high accuracy by using the lesion identification models A1 and A2. Therefore, by using the lesion identification models A1 and A2 and the lesion identification models C1 and C2, it is possible to compensate for a defect of the lesion identification models A1 and A2, and increase identification accuracy for a lesion region.

The lesion detection process may be executed by using the lesion identification models B1 and B2 and the lesion identification models C1 and C2. It may be said that the lesion identification models C1 and C2 cover a special case where a lesion region may not be identified with high accuracy by using the lesion identification models B1 and B2. Therefore, by using the lesion identification models B1 and B2 and the lesion identification models C1 and C2, it is possible to compensate for a defect of the lesion identification models B1 and B2, and increase identification accuracy for a lesion region.

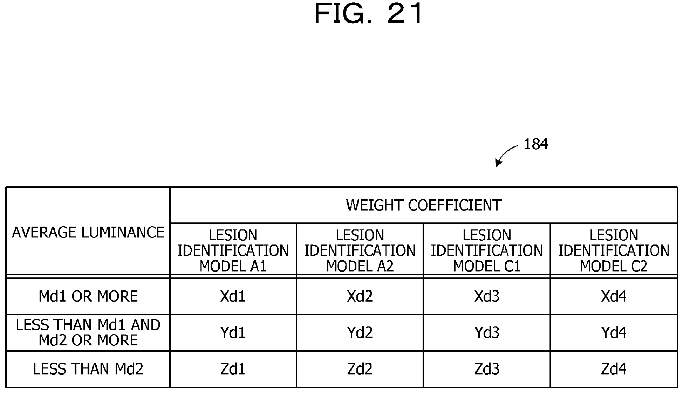

Here, as an example, FIG. 21 illustrates weight coefficients in a case where the lesion identification models A1 and A2 and the lesion identification models C1 and C2 are used.

FIG. 21 is an example of a weight table indicating a correspondence relationship between an image feature amount and a weight coefficient. For example, the detection result output unit 145 may refer to a weight table 184 as illustrated in FIG. 21, and integrate each identification result of the lesion identification models A1, A2, C1, and C2.

As an example in FIG. 21, a region from which a calculated average luminance may be obtained is divided into three regions by using threshold values Md1 and Md2 (where Md1 > Md2). A weight coefficient of each of the lesion identification models A1, A2, C1, and C2 is associated for each of the divided regions.

In a case where the average luminance is equal to or more than the threshold value Md1, Xd1 to Xd4 are applied as the weight coefficients corresponding to the lesion identification models A1, A2, C1, and C2, respectively. In a case where the average luminance is less than the threshold value Md1 and equal to or more than the threshold value Md2, Yd1 to Yd4 are applied as the weight coefficients corresponding to the lesion identification models A1, A2, C1, and C2, respectively. In a case where the average luminance is less than the threshold value Mb2, the weight coefficients Zd1 to Zd4 are applied as the weight coefficients corresponding to the lesion identification models A1, A2, C1, and C2, respectively.

Each of the weight coefficients Xd1 to Xd4, Yd1 to Yd4, and Zd1 to Zd4 takes a value equal to or more than 0 and equal to or less than 1. Xd1 + Xd2 + Xd3 + Xd4 = Yd1 + Yd2 + Yd3 + Yd4 = Zd1 + Zd2 + Zd3 + Zd4 = 1 are set.

As the average luminance is lower, the weight coefficient of the lesion identification model A2 between the lesion identification models A1 and A2 is set to a higher value. For example, Xd1 > Yd1 > Zd1 and Xd2 < Yd2 < Zd2 are set, and Xd1 > Xd2 and Zd1 < Zd2 are assumed. A magnitude relationship between Yd1 and Yd2 may be determined in accordance with setting values of the threshold values Md1 and Md2.

On the other hand, as described above, for the lesion identification models C1 and C2, a weight coefficient when the average luminance is an intermediate value may be set to a high value. For example, Yd3 > Xd3 > Zd3 and Xd4 < Zd4 < Yd4 may be set. A magnitude relationship between Yd3 and Yd4 may be determined in accordance with setting values of the threshold values Md1 and Md2.

By using such a weight coefficient, the detection result output unit 145 may obtain a final lesion identification result by increasing a weight of a probability value by a lesion detection model in which a condition of a CT image used as learning data is close to a condition of an input CT image group among the lesion identification models A1, A2, C1, and C2. As a result, it is possible to accurately detect a lesion region from the simple CT image.

The processing function of the apparatus (for example, the information processing apparatus 1 and the diagnosis support apparatuses 100, 100a, and 100b) described in each of the embodiments described above may be realized by a computer. In such a case, a program describing the processing contents of the functions to be included in each apparatus is provided, and the processing functions described above are realized over the computer by executing the program with the computer. The program describing the processing contents may be recorded in a computer-readable recording medium. The computer-readable recording medium may be a magnetic storage device, an optical disc, a semiconductor memory, or the like. The magnetic storage device may be a hard disk drive (HDD), a magnetic tape, or the like. Examples of the optical disc include a compact disc (CD), a Digital Versatile Disc (DVD), a Blu-ray disc (BD, registered trademark), and the like.

In a case where the program is distributed, for example, a portable-type recording medium such as a DVD or a CD on which the program is recorded is sold. The program may be stored in a storage device of a server computer and transferred from the server computer to another computer via a network.

The computer that executes the program stores, in a storage device thereof, the program recorded on the portable-type recording medium or the program transferred from the server computer, for example. The computer reads the program from the storage device thereof and executes process according to the program. The computer may also read the program directly from the portable-type recording medium and execute the processing according to the program. Each time the program is transferred from the server computer coupled to the computer via the network, the computer may also sequentially execute process according to the received program.

## Claims

1. A lesion detection apparatus (100) comprising:
a memory (102); and
a processor (101) coupled to the memory (102) and configured to execute
a learning process of
classifying a plurality of first tomographic images obtained by imaging an inside of a plurality of first human bodies at predetermined intervals in the height direction of the first human bodies into a plurality of first tomographic image groups on the basis of the degree of accumulation of fat in a specific organ, and
generating a plurality of first lesion identification models for identifying whether or not each unit image region included in a tomographic image as an identification target is a specific lesion region by machine learning which uses each of the plurality of first tomographic image groups as learning data, the unit image region being a pixel or a set of the predetermined number of two or more adjacent pixels; and
a lesion detection process of
calculating, as a first image feature amount, an average luminance value in the region of the specific organ of a plurality of second tomographic images obtained by imaging an inside of a second human body at predetermined intervals in the height direction of the second human bodies,
acquiring a probability that each of the unit image regions included in the plurality of second tomographic images is the specific lesion region from each of the plurality of first lesion identification models, by inputting the plurality of second tomographic images to each of the plurality of first lesion identification models,
determining a weight coefficient corresponding to each of the plurality of first lesion identification models based on the first image feature amount,
calculating, for each of the unit image regions included in the plurality of second tomographic images, an integration value by integrating the probabilities acquired from each of the plurality of first lesion identification models and multiplied by the weight coefficient, and
detecting the specific lesion region from each of the plurality of second tomographic images based on the integration value.

2. The lesion detection apparatus (100) according to claim 1,
wherein
in the calculating of the integration value, the integration value is calculated, and as the average luminance value is lower, a higher weight coefficient is set for the probability from a first lesion identification model generated by using the first tomographic image group which has a higher accumulation degree of fat.

3. The lesion detection apparatus (100) according to claim 1,
wherein the learning process includes a process of
calculating, for each second tomographic image group into which the plurality of first tomographic images are classified for each of the first human bodies, a second image feature amount of the same type as the first image feature amount,
classifying the plurality of first tomographic images into a plurality of third tomographic image groups according to a range of the second image feature amount, and
generating a plurality of second lesion identification models for identifying whether or not each of the unit image regions included in the tomographic image as the identification target is the specific lesion region by machine learning which uses each of the plurality of third tomographic image groups as learning data,
the lesion detection process includes a process of
acquiring the probability for each of the unit image regions included in the plurality of second tomographic images from each of the plurality of second lesion identification models, by inputting the plurality of second tomographic images to each of the plurality of second lesion identification models, and
in the calculating of the integration value,
for each of the unit image regions included in the plurality of second tomographic images, the integration value is calculated by integrating the probabilities acquired from each of the plurality of first lesion identification models and each of the plurality of second lesion identification models, based on the first image feature amount.

4. A lesion detection program for causing a computer to execute:
a learning process of
classifying a plurality of first tomographic images obtained by imaging an inside of a plurality of first human bodies at predetermined intervals in the height direction of the first human bodies into a plurality of first tomographic image groups n the basis of the degree of accumulation of fat in a specific organ, and
generating a plurality of first lesion identification models for identifying whether or not each unit image region included in a tomographic image as an identification target is a specific lesion region by machine learning which uses each of the plurality of first tomographic image groups as learning data, the unit image region being a pixel or a set of the predetermined number of two or more adjacent pixels; and
a lesion detection process of
calculating, as a first image feature amount, an average luminance value in the region of the specific organ of a plurality of second tomographic images obtained by imaging an inside of a second human body at predetermined intervals in the height direction of the second human bodies,
acquiring a probability that each of the unit image regions included in the plurality of second tomographic images is the specific lesion region from each of the plurality of first lesion identification models, by inputting the plurality of second tomographic images to each of the plurality of first lesion identification models,
determining a weight coefficient corresponding to each of the plurality of first lesion identification models based on the first image feature amount,
calculating, for each of the unit image regions included in the plurality of second tomographic images, an integration value by integrating the probabilities acquired from each of the plurality of first lesion identification models and multiplied by the weight coefficient, and
detecting the specific lesion region from each of the plurality of second tomographic images based on the integration value.

## Patentansprüche

1. Läsionsdetektionseinrichtung (100), umfassend:
einen Speicher (102); und
einen Prozessor (101), der mit dem Speicher (102) gekoppelt ist und konfiguriert ist zum Ausführen
eines Lernprozesses des
Klassifizierens einer Vielzahl von ersten tomographischen Bildern, die durch Bildgebung eines Inneren einer Vielzahl von ersten menschlichen Körpern in vorbestimmten Intervallen in der Höhenrichtung der ersten menschlichen Körper erhalten wurden, in eine Vielzahl von ersten tomographischen Bildgruppen auf der Grundlage des Grads der Fetteinlagerung in einem spezifischen Organ, und
Erzeugens einer Vielzahl von ersten Läsionsidentifikationsmodellen zum Identifizieren, ob jeder Bildeinheitsbereich, der in einem tomographischen Bild als Identifikationsziel eingeschlossen ist, ein spezifischer Läsionsbereich ist oder nicht, durch maschinelles Lernen, das jede der Vielzahl von ersten tomographischen Bildgruppen als Lerndaten verwendet, wobei der Bildeinheitsbereich ein Pixel oder eine Menge der vorbestimmten Anzahl von zwei oder mehr benachbarten Pixeln ist; und
eines Läsionsdetektionsprozesses des
Berechnens, als erster Bildmerkmalswert, eines durchschnittlichen Luminanzwerts in dem Bereich des spezifischen Organs einer Vielzahl von zweiten tomographischen Bildern, die durch Bildgebung eines Inneren eines zweiten menschlichen Körpers in vorbestimmten Intervallen in der Höhenrichtung der zweiten menschlichen Körper erhalten wurden,
Erwerbens einer Wahrscheinlichkeit, dass jeder der Bildeinheitsbereiche, der in der Vielzahl von zweiten tomographischen Bildern eingeschlossen ist, der spezifische Läsionsbereich ist, von jedem der Vielzahl von ersten Läsionsidentifikationsmodellen, durch Eingeben der Vielzahl von zweiten tomographischen Bildern in jedes der Vielzahl von ersten Läsionsidentifikationsmodellen,
Bestimmens eines jedem der Vielzahl von ersten Läsionsidentifikationsmodellen entsprechenden Gewichtskoeffizienten basierend auf dem ersten Bildmerkmalswert,
Berechnens, für jeden der Bildeinheitsbereiche, der in der Vielzahl von zweiten tomographischen Bildern eingeschlossen ist, eines Integrationswerts durch Integrieren der Wahrscheinlichkeiten, die von jedem der Vielzahl von ersten Läsionsidentifikationsmodellen erworben wurden und mit dem Gewichtskoeffizienten multipliziert wurden, und
Detektierens des spezifischen Läsionsbereichs aus jedem der Vielzahl von zweiten tomographischen Bildern basierend auf dem Integrationswert.

2. Läsionsdetektionseinrichtung (100) nach Anspruch 1,
wobei
bei dem Berechnen des Integrationswerts der Integrationswert berechnet wird, und wenn der durchschnittliche Luminanzwert niedriger ist, ein höherer Gewichtskoeffizient für die Wahrscheinlichkeit aus einem ersten Läsionsidentifikationsmodell, das unter Verwendung der ersten tomographischen Bildgruppe erzeugt wurde, welche einen höheren Grad der Fetteinlagerung aufweist, festgelegt wird.

3. Läsionsdetektionseinrichtung (100) nach Anspruch 1,
wobei der Lernprozess einen Prozess einschließt, bei dem
für jede zweite tomographische Bildgruppe, in die die Vielzahl von ersten tomographischen Bildern für jeden der ersten menschlichen Körper klassifiziert wird, ein zweiter Bildmerkmalswert desselben Typs wie der erste Bildmerkmalswert berechnet wird,
die Vielzahl von ersten tomographischen Bildern in eine Vielzahl von dritten tomographischen Bildgruppen entsprechend einer Variationsbreite des zweiten Bildmerkmalswerts klassifiziert wird, und
eine Vielzahl von zweiten Läsionsidentifikationsmodellen zum Identifizieren, ob jeder der Bildeinheitsbereiche, der in dem tomographischen Bild als das Identifikationsziel eingeschlossen ist, der spezifische Läsionsbereich ist oder nicht, durch maschinelles Lernen erzeugt wird, das jede der Vielzahl von dritten tomographischen Bildgruppen als Lerndaten verwendet,
der Läsionsdetektionsprozess einen Prozess einschließt, bei dem
die Wahrscheinlichkeit für jeden der Bildeinheitsbereiche, der in der Vielzahl von zweiten tomographischen Bildern eingeschlossen ist, von jedem der Vielzahl von zweiten Läsionsidentifikationsmodellen erworben wird, durch Eingeben der Vielzahl von zweiten tomographischen Bildern in jedes der Vielzahl von zweiten Läsionsidentifikationsmodellen, und
bei dem Berechnen des Integrationswerts
für jeden der Bildeinheitsbereiche, der in der Vielzahl von zweiten tomographischen Bildern eingeschlossen ist, der Integrationswert durch Integrieren der Wahrscheinlichkeiten, die von jedem der Vielzahl von ersten Läsionsidentifikationsmodellen und jedem der Vielzahl von zweiten Läsionsidentifikationsmodellen erworben wurden, basierend auf dem ersten Bildmerkmalswert, berechnet wird.

4. Läsionsdetektionsprogramm zum Veranlassen eines Computers zum Ausführen:
eines Lernprozesses des
Klassifizierens einer Vielzahl von ersten tomographischen Bildern, die durch Bildgebung eines Inneren einer Vielzahl von ersten menschlichen Körpern in vorbestimmten Intervallen in der Höhenrichtung der ersten menschlichen Körper erhalten wurden, in eine Vielzahl von ersten tomographischen Bildgruppen auf der Grundlage des Grads der Fetteinlagerung in einem spezifischen Organ, und
Erzeugens einer Vielzahl von ersten Läsionsidentifikationsmodellen zum Identifizieren, ob jeder Bildeinheitsbereich, der in einem tomographischen Bild als Identifikationsziel eingeschlossen ist, ein spezifischer Läsionsbereich ist oder nicht, durch maschinelles Lernen, das jede der Vielzahl von ersten tomographischen Bildgruppen als Lerndaten verwendet, wobei der Bildeinheitsbereich ein Pixel oder eine Menge der vorbestimmten Anzahl von zwei oder mehr benachbarten Pixeln ist; und
eines Läsionsdetektionsprozesses des
Berechnens, als erster Bildmerkmalswert, eines durchschnittlichen Luminanzwerts in dem Bereich des spezifischen Organs einer Vielzahl von zweiten tomographischen Bildern, die durch Bildgebung eines Inneren eines zweiten menschlichen Körpers in vorbestimmten Intervallen in der Höhenrichtung der zweiten menschlichen Körper erhalten wurden,
Erwerbens einer Wahrscheinlichkeit, dass jeder der Bildeinheitsbereiche, der in der Vielzahl von zweiten tomographischen Bildern eingeschlossen ist, der spezifische Läsionsbereich ist, von jedem der Vielzahl von ersten Läsionsidentifikationsmodellen, durch Eingeben der Vielzahl von zweiten tomographischen Bildern in jedes der Vielzahl von ersten Läsionsidentifikationsmodellen,
Bestimmens eines jeden der Vielzahl von ersten Läsionsidentifikationsmodellen entsprechenden Gewichtskoeffizienten basierend auf dem ersten Bildmerkmalswert,
Berechnens, für jeden der Bildeinheitsbereiche, der in der Vielzahl von zweiten tomographischen Bildern eingeschlossen ist, eines Integrationswerts durch Integrieren der Wahrscheinlichkeiten, die von jedem der Vielzahl von ersten Läsionsidentifikationsmodellen erworben wurden und mit dem Gewichtskoeffizienten multipliziert wurden, und
Detektierens des spezifischen Läsionsbereichs aus jedem der Vielzahl von zweiten tomographischen Bildern basierend auf dem Integrationswert.

## Revendications

1. Appareil (100) de détection de lésion, comprenant :
une mémoire (102) ; et
un processeur (101) relié à la mémoire (102) et configuré pour exécuter
un processus d'apprentissage consistant à
classifier une pluralité de premières images tomographiques obtenues en capturant des images de l'intérieur d'une pluralité de premiers corps humains à des intervalles prédéterminés dans la direction de la hauteur des premiers corps humains, en une pluralité de premiers groupes d'images tomographiques sur la base du degré d'accumulation de graisse dans un organe spécifique, et
générer une pluralité de premiers modèles d'identification de lésion pour identifier si ou non chaque région d'image unitaire incluse dans une image tomographique en tant que cible d'identification est une région de lésion spécifique, par apprentissage automatique qui utilise chacun des premiers groupes d'images tomographiques de la pluralité de premiers groupes d'images tomographiques en tant que données d'apprentissage, la région d'image unitaire étant un pixel ou un ensemble du nombre prédéterminé de deux ou plusieurs pixels adjacents ; et
un processus de détection de lésion consistant à
calculer, en tant que première quantité de caractéristique d'image, une valeur de luminance moyenne dans la région de l'organe spécifique d'une pluralité de deuxièmes images tomographiques obtenues en capturant des images de l'intérieur d'un deuxième corps humain à des intervalles prédéterminés dans la direction de la hauteur des deuxièmes corps humains,
acquérir, à partir de chacun des premiers modèles d'identification de lésion de la pluralité de premiers modèles d'identification de lésion, une probabilité selon laquelle chacune des régions d'image unitaires incluses dans la pluralité de deuxièmes images tomographiques est la région de lésion spécifique, en entrant la pluralité de deuxièmes images tomographiques dans chacun des premiers modèles d'identification de lésion de la pluralité de premiers modèles d'identification de lésion,
déterminer un coefficient de pondération correspondant à chacun des premiers modèles d'identification de lésion de la pluralité de premiers modèles d'identification de lésion sur la base de la première quantité de caractéristique d'image,
calculer, pour chacune des régions d'image unitaires incluses dans la pluralité de deuxièmes images tomographiques, une valeur d'intégration en intégrant les probabilités acquises à partir de chacun des premiers modèles d'identification de lésion de la pluralité de premiers modèles d'identification de lésion et multipliées par le coefficient de pondération, et
détecter la région de lésion spécifique à partir de chacune des deuxièmes images tomographiques de la pluralité de deuxièmes images tomographiques sur la base de la valeur d'intégration.

2. Appareil (100) de détection de lésion selon la revendication 1,
dans lequel,
dans le calcul de la valeur d'intégration, la valeur d'intégration est calculée, et, au fur et à mesure que la valeur de luminance moyenne baisse, un coefficient de pondération supérieur est défini pour la probabilité provenant d'un premier modèle d'identification de lésion généré en utilisant le premier groupe d'images tomographiques qui présente un degré d'accumulation de graisse supérieur.

3. Appareil (100) de détection de lésion selon la revendication 1,
dans lequel le processus d'apprentissage inclut un processus consistant à
calculer, pour chaque deuxième groupe d'images tomographiques dans lequel les premières images tomographiques de la pluralité de premières images tomographiques sont classifiées pour chacun des premiers corps humains, une deuxième quantité de caractéristique d'image du même type que la première quantité de caractéristique d'image,
classifier la pluralité de premières images tomographiques en une pluralité de troisièmes groupes d'images tomographiques selon une plage de la deuxième quantité de caractéristique d'image, et
générer une pluralité de deuxièmes modèles d'identification de lésion pour identifier si ou non chacune des régions d'image unitaires incluses dans l'image tomographique en tant que cible d'identification est la région de lésion spécifique, par apprentissage automatique qui utilise chacun des troisièmes groupes d'images tomographiques de la pluralité de troisièmes groupes d'images tomographiques en tant que données d'apprentissage,
le processus de détection de lésion inclut un processus consistant à
acquérir, à partir de chacun des deuxièmes modèles d'identification de lésion de la pluralité de deuxièmes modèles d'identification de lésion, la probabilité pour chacune des régions d'image unitaires incluses dans la pluralité de deuxièmes images tomographiques, en entrant la pluralité de deuxièmes images tomographiques dans chacun des deuxièmes modèles d'identification de lésion de la pluralité de deuxièmes modèles d'identification de lésion, et,
dans le calcul de la valeur d'intégration,
pour chacune des régions d'image unitaires incluses dans la pluralité de deuxièmes images tomographiques, la valeur d'intégration est calculée en intégrant les probabilités acquises à partir de chacun des premiers modèles d'identification de lésion de la pluralité de premiers modèles d'identification de lésion et de chacun des deuxièmes modèles d'identification de lésion de la pluralité de deuxièmes modèles d'identification de lésion, sur la base de la première quantité de caractéristique d'image.

4. Programme de détection de lésion pour amener un ordinateur à exécuter :
un processus d'apprentissage consistant à
classifier une pluralité de premières images tomographiques obtenues en capturant des images de l'intérieur d'une pluralité de premiers corps humains à des intervalles prédéterminés dans la direction de la hauteur des premiers corps humains en une pluralité de premiers groupes d'images tomographiques sur la base du degré d'accumulation de graisse dans un organe spécifique, et
générer une pluralité de premiers modèles d'identification de lésion pour identifier si ou non chaque région d'image unitaire incluse dans une image tomographique en tant que cible d'identification est une région de lésion spécifique, par apprentissage automatique qui utilise chacun des premiers groupes d'images tomographiques de la pluralité de premiers groupes d'images tomographiques en tant que données d'apprentissage, la région d'image unitaire étant un pixel ou un ensemble du nombre prédéterminé de deux ou plusieurs pixels adjacents ; et
un processus de détection de lésion consistant à
calculer, en tant que première quantité de caractéristique d'image, une valeur de luminance moyenne dans la région de l'organe spécifique d'une pluralité de deuxièmes images tomographiques obtenues en capturant des images de l'intérieur d'un deuxième corps humain à des intervalles prédéterminés dans la direction de la hauteur des deuxièmes corps humains,
acquérir une probabilité selon laquelle chacune des régions d'image unitaires incluses dans la pluralité de deuxièmes images tomographiques est la région de lésion spécifique à partir de chacun des premiers modèles d'identification de lésion de la pluralité de premiers modèles d'identification de lésion, en entrant la pluralité de deuxièmes images tomographiques dans chacun des premiers modèles d'identification de lésion de la pluralité de premiers modèles d'identification de lésion,
déterminer un coefficient de pondération correspondant à chacun des premiers modèles d'identification de lésion de la pluralité de premiers modèles d'identification de lésion sur la base de la première quantité de caractéristique d'image,
calculer, pour chacune des régions d'image unitaires incluses dans la pluralité de deuxièmes images tomographiques, une valeur d'intégration en intégrant les probabilités acquises à partir de chacun des premiers modèles d'identification de lésion de la pluralité de premiers modèles d'identification de lésion et multipliées par le coefficient de pondération, et
détecter la région de lésion spécifique à partir de chacune des deuxièmes images tomographiques de la pluralité de deuxièmes images tomographiques sur la base de la valeur d'intégration.
